# EUROPEAN PATENT APPLICATION

(11) **EP 4 786 489 A1**
(43) Date of publication of application: **05.08.2026**
(21) Application number: 24870923.0
(22) Date of filing: 27.09.2024
(51) Int. Cl.: C07K 14/54, A61K 38/20, C07K 17/08

(54) **IL-22 MUTANT, PREPARATION METHOD THEREFOR, AND USE THEREOF**

(30) Priority: 28.09.2023 CN 202311272942
(71) Applicant: Nanjing Novoacine Biotechnology Co., Ltd., Nanjing, Jiangsu 210032 (CN)
(72) Inventor: ZHONG, Kai, Nanjing, Jiangsu 210032 (CN); WU, Wei, Nanjing, Jiangsu 210032 (CN); QI, Xin, Nanjing, Jiangsu 210032 (CN); XIONG, Xinhui, Nanjing, Jiangsu 210032 (CN); ZHANG, Tao, Nanjing, Jiangsu 210032 (CN); WANG, Qing, Nanjing, Jiangsu 210032 (CN); LI, Xing, Nanjing, Jiangsu 210032 (CN); YE, Yi, Nanjing, Jiangsu 210032 (CN); DOU, Chunfang, Nanjing, Jiangsu 210032 (CN)
(74) Representative: Bianchetti & Minoja with Trevisan & Cuonzo IPS SRL
(86) International application number: PCT/CN2024/121613
(87) International publication number: WO 2025/067385

(57) **Abstract**

An IL-22 mutant, a preparation method therefor, and a use thereof, relating to the technical field of biology. By mutating IL-22 and modifying the mutation site with PEG, the obtained mutant has good STAT3/STAT1 preference, significantly reduced STAT1 signal activation, and significantly reduced inflammatory effects. The IL-22 mutant can preferentially act on intestinal target organs in vivo and is not prone to activating skin cells to produce adverse reactions.

## Description

This application claims priority to Chinese Patent Application No. 202311272942.0, filed on September 28, 2023, entitled "IL-22 mutants, preparation methods and applications thereof", which is incorporated herein by reference in its entirety.

### FIELD OF THE INVENTION

This invention relates to the field of biotechnology, and in particular to IL-22 mutants, preparation methods and applications thereof.

### BACKGROUND OF THE INVENTION

IL-22 is a member of the interleukin-10 family and has a variety of biological functions, playing a key role in maintaining tissue homeostasis during inflammatory infections. IL-22 is produced by various immune cells at physiological barrier interfaces, including CD4⁺ T cells and ILC3s, and acts on epithelial cells, where it enhances tissue integrity and promotes tissue repair and regeneration. IL-22 can exert its effects in multiple organs, including the pancreas, gastrointestinal tract, and liver. Studies have demonstrated that IL-22 exerts significant protective effects in multiple mouse disease models, including models of IBD, acute liver injury, and GvHD-induced intestinal damage. IL-22 maintains intestinal structural integrity by inducing the expression of genes related to proliferation, wound healing, and apoptosis. In addition, IL-22 can induce the expression of innate antimicrobial molecules, including regenerating islet-derived (Reg) proteins, defensins, and S100 proteins, thereby supporting intestinal barrier function. Therefore, Reg3 molecules (Reg3A in humans and Reg3β in mice), as important pharmacodynamic (PD) biomarkers of IL-22 in the treatment of intestinal diseases, have been widely used in various studies.

In addition to its organ-protective functions, IL-22 also has potential pathogenic effects. IL-22 can activate inflammatory Th17 cells and exhibit pro-inflammatory activities in the skin, liver, and gastrointestinal tract, leading to the expression of acute-phase response proteins such as SAA-1/2. Both animal experiments and human studies have shown that IL-22 administration can significantly increase serum levels of the acute-phase response protein SAA. Therefore, SAA is commonly used as an important inflammation-related biomarker to evaluate the pro-inflammatory effects of IL-22 in both nonclinical and clinical studies.

IL-22 activates downstream signaling pathways by binding to a heterodimeric receptor complex (IL-22Rα/IL-10Rβ). IL-10Rβ is widely distributed, while the expression of IL-22Rα is mainly limited to epithelial cells, which determines the cell specificity of IL-22. Upon receptor engagement, IL-22 primarily activates signal transducer and activator of transcription 1(STAT1) and STAT3. The literature indicates that the expression of genes associated with organ protection and tissue regeneration is mainly mediated by STAT3, whereas activation of STAT1 is associated with various pro-inflammatory effects. Accordingly, IL-22 molecules with signaling activation preference can be obtained, which are capable of substantially reducing pro-inflammatory effects while maintaining their organ-protective activity. Such molecules represent an important research direction in IL-22-related therapeutic strategies.

In addition, the expression abundance of IL-10Rβ varies markedly among different tissues and cell types, resulting in differential STAT3 activation by IL-22 in distinct tissues and cells. It has been reported in the literature that IL-22 mutants, through amino acid mutations that alter their affinity for IL-10Rβ, are able to better retain STAT3 activity in intestinal cells with relatively high IL-10Rβ expression, while reducing STAT3 activity in cells with lower IL-10Rβ expression, such as skin cells, thereby exhibiting the potential to reduce skin-related toxicity.

In certain prior art, the half-life of IL-22 was markedly extended by fusing the IL-22 molecule to an Fc domain. However, Fc fusion does not affect the activation preference, that still exhibits pronounced pro-inflammatory activity and off-target organ toxicity. Literature Results have shown that IL-22-Fc (UTTR14147A) has significant pro-inflammatory effects both *in vivo* and *in vitro*. Phase I clinical results indicate that UTTR14147A induces significant elevations in serum SAA and CRP from moderate to high doses, accompanied by notable cutaneous adverse reactions manifesting as dry skin, desquamation, and erythema.

In other prior art, STAT1 activation was reduced and the preference was enhanced through multi-site mutations. However, IL-22 mutants exhibit a relatively short half-life, similar to that of native IL-22, thereby presenting significant limitations for clinical application. By comparing wt IL-22 and IL-22-Fc, it has been found that the fusion protein displays markedly lower activity than the prototype protein, suggesting substantial uncertainty in the half-life extension modification of IL-22 mutants.

Accordingly, there remains an urgent need in the art to improve the preference of IL-22 while reducing its adverse effects.

### SUMMARY OF THE INVENTION

In view of this, the technical problem to be solved by the present invention is to provide an IL22 mutant with signaling activation preference, its preparation method and application.

The present invention provides an IL-22 mutant, wherein at least one amino acid residue within positions 10 to 95 of the amino acid sequence set forth in SEQ ID NO: 1 is substituted with cysteine-PEG or p-acetyl-L-phenylalanine-PEG.

In certain embodiments of the present invention, the IL-22 mutant comprises one, two, or more than two mutation sites, and the present invention is not limited thereto. In some embodiments, the mutation site is at least one of the following positions: position 10, position 12, position 15, position 16, position 17, position 18, position 21, position 22, position 64, position 72, position 75, position 76, position 79, position 83, position 84, position 86, position 87, position 88, position 91, or position 95.

In some embodiments, the mutation site is any one of the following positions: position 10, position 12, position 15, position 16, position 17, position 18, position 21, position 22, position 64, position 72, position 75, position 76, position 79, position 83, position 84, position 86, position 87, position 88, position 91, or position 95.

In some specific embodiments, the mutation sites are: D10pAcF, S12pAcF, Q15pAcF, Q16pAcF, P17pAcF, Y18pAcF, N21pAcF, R22pAcF, N64pAcF, F72pAcF, S75pAcF, D76pAcF, Q79pAcF, Q83pAcF, E84pAcF, V86pAcF, P8 pAcF, F88pAcF, R91pAcF, R95pAcF. Alternatively, the mutation site may be P17C, D76C, P87C, or F22C.

In the present invention, human interleukin-22 (wt IL-22, as shown in SEQ ID NO: 1) is used as the starting molecule. Mutations are introduced at different positions to incorporate a non-natural amino acid (pAcF) or cysteine (Cys), followed by site-specific modification of polyethylene glycol at the mutated positions. The polyethylene glycol conjugation site and the molecular weight of PEG are further optimized to obtain a site-directed PEGylated interleukin-22 exhibiting strong preference and improved *in vivo* efficacy. PEG30 indicates a PEG with a molecular weight of 30K.

Among them, cysteine-PEG refers to cysteine conjugated to PEG, wherein the maleimide group of PEG reacts with the thiol group of cysteine to form a thioether linkage. p-acetyl-L-phenylalanine-PEG refers to p-acetyl-L-phenylalanine conjugated to PEG, wherein the hydroxylamine group of PEG reacts with the carbonyl group of p-acetyl-L-phenylalanine to form an oxime linkage. In certain embodiments of the present invention, the molecular weight of PEG ranges from 30 kDa to 80 kDa. For example, PEG has a molecular weight of 30kD, 40kD, 50kD, 60kD, 70kD or 80kD.

In certain embodiments, the mutant of the present invention is as follows:
Asp at position 10 of the amino acid sequence set forth in SEQ ID NO: 1 is replaced with p-acetyl-L-phenylalanine-PEG30;
Ser at position 12 of the amino acid sequence set forth in SEQ ID NO: 1 is replaced with p-acetyl-L-phenylalanine-PEG30;
Gln at position 15 of the amino acid sequence set forth in SEQ ID NO: 1 is replaced with p-acetyl-L-phenylalanine-PEG30;
Gln at position 16 of the amino acid sequence set forth in SEQ ID NO: 1 is replaced with p-acetyl-L-phenylalanine-PEG30;
Pro at position 17 of the amino acid sequence set forth in SEQ ID NO: 1 is replaced with p-acetyl-L-phenylalanine-PEG30;
Pro at position 17 of the amino acid sequence set forth in SEQ ID NO: 1 is replaced with p-acetyl-L-phenylalanine-PEG40;
Pro at position 17 of the amino acid sequence set forth in SEQ ID NO: 1 is replaced with cysteine-PEG40;
Pro at position 17 of the amino acid sequence set forth in SEQ ID NO: 1 is replaced with cysteine-PEG60;
Pro at position 17 of the amino acid sequence set forth in SEQ ID NO: 1 is replaced with cysteine-PEG80;
Tyr at position 18 of the amino acid sequence set forth in SEQ ID NO: 1 is replaced with p-acetyl-L-phenylalanine-PEG30;
Asn at position 21 of the amino acid sequence set forth in SEQ ID NO: 1 is replaced with p-acetyl-L-phenylalanine-PEG30;
Arg at position 22 of the amino acid sequence set forth in SEQ ID NO: 1 is replaced with p-acetyl-L-phenylalanine-PEG30;
Asn at position 64 of the amino acid sequence set forth in SEQ ID NO: 1 is replaced with p-acetyl-L-phenylalanine-PEG30;
Phe at position 72 of the amino acid sequence set forth in SEQ ID NO: 1 is replaced with p-acetyl-L-phenylalanine-PEG30;
Ser at position 75 of the amino acid sequence set forth in SEQ ID NO: 1 is replaced with p-acetyl-L-phenylalanine-PEG30;
Asp at position 76 of the amino acid sequence set forth in SEQ ID NO: 1 is replaced with p-acetyl-L-phenylalanine-PEG30;
Asp at position 76 of the amino acid sequence set forth in SEQ ID NO: 1 is replaced with cysteine-PEG40;
Asp at position 76 of the amino acid sequence set forth in SEQ ID NO: 1 is replaced with cysteine-PEG60;
Asp at position 76 of the amino acid sequence set forth in SEQ ID NO: 1 is replaced with cysteine-PEG80;
Gln at position 79 of the amino acid sequence set forth in SEQ ID NO: 1 is replaced with p-acetyl-L-phenylalanine-PEG30;
Gln at position 83 of the amino acid sequence set forth in SEQ ID NO: 1 is replaced with p-acetyl-L-phenylalanine-PEG30;
Glu at position 84 of the amino acid sequence set forth in SEQ ID NO: 1 is replaced with p-acetyl-L-phenylalanine-PEG30;
Val at position 86 of the amino acid sequence set forth in SEQ ID NO: 1 is replaced with p-acetyl-L-phenylalanine-PEG30;
Pro at position 87 of the amino acid sequence set forth in SEQ ID NO: 1 is replaced with p-acetyl-L-phenylalanine-PEG30;
Pro at position 87 of the amino acid sequence set forth in SEQ ID NO: 1 is replaced with p-acetyl-L-phenylalanine-PEG40;
Pro at position 87 of the amino acid sequence set forth in SEQ ID NO: 1 is replaced with cysteine-PEG40;
Pro at position 87 of the amino acid sequence set forth in SEQ ID NO: 1 is replaced with cysteine-PEG60;
Pro at position 87 of the amino acid sequence set forth in SEQ ID NO: 1 is replaced with cysteine-PEG80;
Phe at position 88 of the amino acid sequence set forth in SEQ ID NO: 1 is replaced with p-acetyl-L-phenylalanine-PEG30;
Phe at position 88 of the amino acid sequence set forth in SEQ ID NO: 1 is replaced with p-acetyl-L-phenylalanine-PEG40;
Arg at position 91 of the amino acid sequence set forth in SEQ ID NO: 1 is replaced with p-acetyl-L-phenylalanine-PEG30;
Arg at position 95 of the amino acid sequence set forth in SEQ ID NO: 1 is replaced with p-acetyl-L-phenylalanine-PEG30.

In some embodiments of the present invention, for ease of purification, a tag is further linked to the terminus of the mutant. For example, a 6×His tag is fused to the C-terminus of the IL-22 mutant.

The site-directed PEGylated interleukin-22 with activation preference provided by this invention, compared with IL-22-Fc, can increase STAT3/STAT1 activation preference, significantly reduce STAT1 signal activation, and significantly reduce pro-inflammatory effects. Furthermore, it can enhance tissue preference by reducing activation of cells with low IL-10Rβ abundance, such as skin cells, thereby having the potential to reduce skin-related adverse effects.

The present invention further provides a biological material selected from any one of the following items I) -V):
I) A nucleic acid encoding the IL-22 mutant as described above;
II) An expression cassette comprising the nucleic acids of I);
III) An expression vector comprising the nucleic acid of I) or the expression cassette of II);
IV) A host comprising the expression vector of III); and
V) A host in which the nucleic acid of I) or the expression cassette of II) is integrated into the genome.

Furthermore, in the present invention, the method for preparing the IL-22 mutant comprises:
replacing at least one amino acid within positions 10-95 of the amino acid sequence shown in SEQ ID NO:1 with p-acetyl-L-phenylalanine, and reacting the resulting protein with PEG-hydroxylamine to obtain the IL-22 mutant; or
replacing at least one amino acid within positions 10-95 of the amino acid sequence shown in SEQ ID NO:1 with cysteine, and reacting the resulting protein with PEG-maleimide to obtain the IL-22 mutant.

In the present invention, the catalyst for the reaction with PEG-hydroxylamine is ammonium chloride and acetylhydrazine.

In the present invention, the molar ratio of PEG-hydroxylamine, ammonium chloride, acetylhydrazine, and the IL-22 mutant is 125:30:5:1; and the molar ratio of PEG-maleimide to the IL-22 mutant is 5:1.

Specifically, the method for replacing at least one amino acid within positions 10-95 of the amino acid sequence shown in SEQ ID NO:1 with p-acetyl-L-phenylalanine comprises: constructing an expression vector encoding the IL-22 mutant, co-transforming a host with the expression vector and an auxiliary plasmid capable of incorporating pAcF, and expressing the mutant protein. In the expression vector, within the nucleic acid encoding the IL-22 mutant, the codon at the mutation site is replaced with an amber codon.

Specifically, the method for replacing at least one amino acid within positions 10-95 of the amino acid sequence shown in SEQ ID NO:1 with cysteine comprises: constructing an expression vector encoding the IL-22 mutant, transforming a host with the expression vector, and expressing the mutant protein. In the expression vector, within the nucleic acid encoding the IL-22 mutant, the codon at the mutation site is replaced with a Cys codon.

Furthermore, the present invention also provides the use of the IL-22 mutant in the preparation of a medicament for treating immune-related diseases.

In the present invention, the immune-related diseases include rheumatoid arthritis, insulin-dependent diabetes mellitus, atherosclerosis, hemolytic anemia, rheumatic fever, thyroiditis, Crohn's disease, myasthenia gravis, glomerulonephritis, hepatitis and fatty liver disease, multiple sclerosis, alopecia areata, psoriasis, vitiligo, dystrophic epidermolysis bullosa, systemic lupus erythematosus, graft-versus-host disease, ulcerative colitis, pancreatitis, psoriatic arthritis, and diabetic foot ulcer.

In the present invention, the treatment comprises improving STAT3/STAT1 activation preference and/or improving tissue activation preference. The improvement of STAT3/STAT1 activation preference includes increasing STAT3-mediated physiological activity and/or decreasing STAT1-mediated physiological activity. The STAT3-mediated physiological activity includes the expression of organ protection- and regeneration-related genes (e.g., Reg3 and Muc1), whereas the STAT1-mediated physiological activity includes various pro-inflammatory effects (e.g., induction of pro-inflammatory CXCL1 expression). The improvement of tissue activation preference refers to preferentially exerting tissue-protective effects in target organs such as the intestine and/or not inducing or reducing inflammatory effects in non-target organs such as the skin.

Furthermore, the present invention also provides a pharmaceutical composition comprising the IL-22 mutant. The pharmaceutical composition of the present invention also comprises pharmaceutically acceptable excipients. In the present invention, the pharmaceutical dosage form is an oral formulation or an injectable formulation, preferably an injectable formulation, which includes an injection solution or a lyophilized powder for injection. The pharmaceutically acceptable excipients include pH adjusters, osmotic pressure regulators, lyophilization protectants, or solvents.

The pharmaceutical composition of the present invention may further comprise other anti-inflammatory agents or immunomodulatory drugs, including but not limited to: local or oral corticosteroids, such as prednisolone, methylprednisolone, and budesonide; aminosalicylates, such as mesalazine and olsalazine; immunomodulators, such as azathioprine, 6-mercaptopurine, methotrexate, cyclosporine, IL-10, IL-11, IL-12, CD74 antagonists, CD40 antagonists, OX40L antagonists, GM-CSF, pimecrolimus, or rapamycin; anti-TNFα agents, such as infliximab, adalimumab, onercept, and etanercept; IL-12/IL-23 antagonists, such as ustekinumab, guselkumab, and risankizumab; anti-inflammatory agents, such as PDE-4 inhibitors, TACE inhibitors, ICE inhibitors, and IL-2 receptor antagonists, such as daclizumab; selective adhesion molecule antagonists, such as natalizumab; analgesics, including but not limited to COX-2 inhibitors, such as rofecoxib and celecoxib; P/Q-type voltage-sensitive channel modulators, such as gabapentin and pregabalin; NK-1 receptor antagonists; cannabinoid receptor modulators; and anti-cancer, anti-tumor, anti-angiogenic agents, or chemotherapeutic drugs. Such additional agents may be included in the same composition or administered separately.

Furthermore, the present invention also provides a method for treating an immune-related disease, comprising administering the pharmaceutical of the present invention. The method of administration of the pharmaceutical includes oral administration or injection, preferably injection, wherein the injection includes intramuscular injection or intravenous injection.

Furthermore, the present invention also provides a method for altering IL-22 receptor activation preference while reducing the toxicity of the IL-22 mutant, comprising:
substituting the amino acid at position 76 and/or position 87 of IL-22 as shown in SEQ ID NO:1 with p-acetyl-L-phenylalanine or cysteine; and
site-specifically PEGylating the amino acid at position 76 and/or position 87 to obtain the IL-22 mutant.

In the method, the average molecular weight of the PEG ranges from 30kD to 80kD, preferably ranges from 30kD to 60kD.

In the present invention, IL-22 is mutated and PEG is conjugated at the mutation site. The resulting mutant exhibits favorable STAT3/STAT1 preference, with significantly reduced STAT1 signaling activation and thus a markedly decreased pro-inflammatory effect. Moreover, *in vivo*, the mutant preferentially acts on the intestinal target organ and is less likely to activate skin cells, thereby reducing adverse effects.

### DESCRIPTION OF THE DRAWINGS

Figure 1 shows the activity of PEGylated IL-22 non-natural amino acid mutants in activating a STAT3 reporter gene at different concentrations.
Figure 2 shows the activity of PEGylated IL-22 non-natural amino acid mutants in activating STAT3 phosphorylation in Colo205 cells at different concentrations, as detected by ELISA.
Figure 3 shows the activity of PEGylated IL-22 non-natural amino acid mutants in activating STAT1 phosphorylation in Colo205 cells at different concentrations, as detected by ELISA.
Figure 4 shows the activity of PEGylated IL-22 non-natural amino acid mutants in activating STAT3 phosphorylation in HT29 cells at different concentrations, as detected by FACS.
Figure 5 shows the activity of PEGylated IL-22 non-natural amino acid mutants in activating STAT1 phosphorylation in HT29 cells at different concentrations, as detected by FACS.
Figure 6 shows the activity of PEGylated IL-22 cysteine mutants in activating STAT3 phosphorylation in HT29 cells at different concentrations, as detected by FACS.
Figure 7 shows the activity of PEGylated IL-22 cysteine mutants in activating STAT1 phosphorylation in HT29 cells at different concentrations, as detected by FACS.
Figure 8 shows the activity of PEGylated IL-22 mutants in activating STAT3 phosphorylation in HaCaT cells at different concentrations, as detected by FACS.
Figure 9 shows the plasma concentration-time curve of Reg3β in mice following a single administration of PEGylated IL-22 mutants.
Figure 10 shows the plasma concentration-time curve of Reg3β in mice following a single administration of PEGylated IL-22 mutants.
Figure 11 shows the plasma concentration-time curve of SAA in mice following a single administration of PEGylated IL-22 mutants.
Figure 12 shows the pharmacokinetic curve in mice following a single administration of PEGylated IL-22 mutants.
Figure 13 shows the body weight change curve after drug administration in a DSS-induced colitis mouse efficacy model.
Figure 14 shows the DAI scores after drug administration in a DSS-induced colitis mouse efficacy model.
Figure 15 shows the colon length at the study endpoint of following drug administration in a DSS-induced colitis mouse efficacy model.
Figure 16 shows the colon histological score at the study endpoint of following drug administration in a DSS-induced colitis mouse efficacy model.
Figure 17 shows the colon histopathological section (G1) at the study endpoint of following drug administration in a DSS-induced colitis mouse efficacy model.
Figure 18 shows the colon histopathological section (G4) at the study endpoint of following drug administration in a DSS-induced colitis mouse efficacy model.
Figure 19 shows the ear thickness increase curve in mice.

### DETAILED DESCRIPTION OF THE INVENTION

The present invention provides IL-22 mutants, as well as methods for their preparation and use. Those skilled in the art may refer to the teachings herein and appropriately modify the process parameters to implement the invention. It should be particularly noted that all similar substitutions and modifications that would be apparent to those skilled in the art are intended to be encompassed within the scope of the present invention. The methods and applications of the present invention have been described with reference to preferred embodiments. It will be apparent to those skilled in the art that various modifications, variations, and combinations of the methods and applications described herein may be made without departing from the content, spirit, and scope of the present invention, and such modifications are intended to fall within the scope of the present invention.

Unless otherwise defined herein, scientific and technical terms used in connection with the present invention shall have the meanings commonly understood by one of ordinary skill in the art.

In the present application, the term "and/or" describes an associative relationship between related objects and indicates that three situations may exist. For example, "A and/or B" may refer to the presence of A alone, the presence of both A and B, or the presence of B alone. A and B may each be singular or plural.

As used herein, "at least one" means one or more, and "a plurality of" means two or more. "At least one of the following items" or similar expressions refer to any combination of the listed items, including any single item or any combination of multiple items.

The terms "comprise," "include," and "have" are used interchangeably herein to indicate an open-ended and inclusive meaning, signifying that additional elements not expressly listed may be present. It should also be understood that use of the terms "comprise," "include," and "have" herein also provides support for embodiments described as "consisting of."

As used herein, the term "nucleic acid" refers to a nucleic acid encoding IL-22, which may be single-stranded or double-stranded, and may be DNA, RNA, cDNA, or PNA. The nucleic acid may comprise nucleotide sequences having different functions, such as coding regions and non-coding regions such as regulatory sequences (e.g., promoters or transcription terminators). The nucleic acid may be linear or circular in topology. It may be, for example, part of a vector (such as an expression vector or cloning vector), or a fragment or a combination of multiple fragments. The nucleic acid may be obtained directly from a natural source, or may be prepared with the assistance of recombinant, enzymatic, or chemical techniques. The RNA form may be mRNA transcribed from a gene. The nucleic acid sequence described in the present invention may be a wild-type sequence, or may be codon-optimized according to the host. The present invention does not limit the sequence in this regard, and all such embodiments fall within the scope of protection of the present invention.

As used herein, the term "expression cassette" comprises the nucleic acid described in the present invention together with a promoter and a terminator. In order to regulate the level of expression or protein folding, the expression cassette may further comprise additional elements.

As used herein, the term "vector" refers to a nucleic acid molecule capable of amplifying another nucleic acid operably linked thereto. The term includes vectors that exist as self-replicating nucleic acid constructs, as well as vectors that integrate into the genome of a host cell into which they have been introduced. Certain vectors are capable of directing the expression of nucleic acids operably linked thereto. Such vectors are referred to herein as "expression vectors." In the present invention, the vector is used for amplification and preservation of the nucleic acid, or for expression of the mutant in a host. The vector described in the present invention comprises a backbone and the nucleic acid. Specifically, the backbone may be derived from plants, animals, bacteria, fungi, bacteriophages, or viruses, and the present invention is not limited in this regard.

As used herein, the term "host cell" refers to a cell into which an exogenous nucleic acid has been introduced, and includes the progeny of such a cell. Host cells include "transformants" and "transformed cells," encompassing the originally transformed cells and their progenies, regardless of the number of passages. The progenies may not be completely identical to the parental cell in nucleic acid content and may contain mutations. Included herein are mutant progeny that possess the same function or biological activity as that for which the originally transformed cell was screened or selected. In the embodiments of the present invention, a prokaryotic expression system is employed, particularly using Escherichia coli as an example for expressing the mutant. In addition, other suitable expression systems, such as yeast expression systems or insect cell expression systems, may also be used for preparation of the mutant, and the present invention is not limited in this regard.

As used herein, the term "treatment" refers to surgical or therapeutic treatment intended to prevent or alleviate (reduce) undesirable physiological changes or pathological conditions in a subject, such as cancer or tumors. Beneficial or desired clinical outcomes include, but are not limited to, alleviation of symptoms, reduction in disease severity, stabilization of disease state (i.e., no worsening), delay or slowing of disease progression, improvement or remission of disease condition, and remission (whether partial or complete), whether detectable or undetectable. Subjects in need of treatment include those already suffering from a disease or disorder, those susceptible to developing a disease or disorder, and those intending to prevent a disease or disorder. When terms such as slowing, alleviating, reducing, ameliorating, or relieving are used, they also encompass elimination, disappearance, or prevention of occurrence.

As used herein, the term subject of "administration" refers to a living organism that receives treatment for a specific disease or condition as described in the present invention. Exemplary subjects receiving treatment for a disease or condition is a mammal, such as a human, a primate (e.g., monkey), or a non-primate mammal.

It should be understood that, in various embodiments of the present application, the numbering of the above processes does not imply a specific order of execution. Some or all of the steps may be performed in parallel or sequentially. The execution order of each process should be determined by its function and inherent logic, and should not be construed as limiting the implementation of the embodiments of the present application.

The present invention relates to sequences comprising:
Amino acid sequence of human IL22: 
APISSHCRLDKSNFQQPYITNRTFMLAKEASLADNNTDVRLIGEKLFHGVSMS
ERCYLMKQVLNFTLEEVLFPQSDRFQPYMQEVVPFLARLSNRLSTCHIEGDDLHIQ
RNVQKLKDTVKKLGESGEIKAIGELDLLFMSLRNACI (denoted as wt IL-22 in the
examples, with the sequence shown in SEQ ID NO.1)
Amino acid sequence of the control IL22 mutant hB3-his (The underlined amino acids indicate the mutated amino acids): 
APISSHCRLDKENFQQPYITNRTFMLAKEASLADNNTDVRLIGEKLFHGVSMS
ERCYLMKQVLNFTLEEVLFPQSDRFQPYMAEVVPFLAALSNALSTCHIEGDDLHIQ
RNVQKLKDTVKKLGESGEIKAIGELDLLFMSLRNACIHHHHHH (denoted as hB3-his
in the examples, with the sequence shown in SEQ ID NO: 2)
Amino acid sequence of IL-22-Fc: 
APISSHCRLDKSNFQQPYITNRTFMLAKEASLADNNTDVRLIGEKLFHGVSMS
ERCYLMKQVLNFTLEEVLFPQSDRFQPYMQEVVPFLARLSNRLSTCHIEGDDLHIQ
RNVQKLKDTVKKLGESGEIKAIGELDLLFMSLRNACIRVESKYGPPCPPCPAPEFLG
GPSVFLFPPKPKDTLMISRTPEVTCVVVDVSQEDPEVQFNWYVDGVEVHNAKTKP
REEQFGSTYRVVSVLTVLHQDWLNGKEYKCKVSNKGLPSSIEKTISKAKGQPREPQ
VYTLPPSQEEMTKNQVSLTCLVKGFYPSDIAVEWESNGQPENNYKTTPPVLDSDGS
FFLYSRLTVDKSRWQEGNVFSCSVMHEALHNHYTQKSLSLSLGK (SEQ ID NO: 3)
Nucleic acid sequence of wild-type IL-22 codon-optimized for Escherichia coli: 
GCTCCCATAAGTTCACACTGTAGGCTAGATAAGTCCAACTTCCAACAGCCGT
ACATTACCAATCGCACGTTTATGCTGGCGAAAGAAGCGAGCTTGGCGGACAACA
ACACCGATGTTCGTCTTATCGGCGAGAAGTTGTTTCATGGTGTGAGCATGTCTGA
ACGCTGCTATCTGATGAAACAGGTGTTGAATTTCACTCTGGAAGAAGTTCTGTT
CCCGCAGAGCGATCGTTTTCAACCATACATGCAAGAAGTCGTGCCGTTTCTGGC
TCGTTTATCTAACCGCCTGTCGACCTGTCATATCGAGGGCGACGACCTGCATATC
CAGCGTAATGTTCAGAAACTGAAGGACACCGTAAAAAAGCTGGGTGAGAGCGG
TGAGATCAAAGCAATTGGCGAGCTGGATTTGCTGTTCATGTCGCTGCGTAACGC
CTGCATT (SEQ ID NO: 4).
Nucleic acid sequence of hB3-his codon-optimized for Escherichia coli:

GCTCCCATAAGTTCACACTGTAGGCTAGATAAGGAGAACTTCCAACAACCA
TACATCACGAACCGTACCTTTATGCTGGCGAAAGAAGCTAGCTTGGCGGATAAC
AACACCGATGTTCGTCTGATCGGCGAGAAATTATTCCACGGCGTGAGCATGTCT
GAACGCTGCTATCTGATGAAACAGGTTTTGAATTTCACCCTCGAAGAGGTCCTG
TTTCCGCAAAGCGATCGTTTTCAGCCGTACATGGCAGAAGTGGTGCCGTTTCTG
GCGGCACTGTCAAATGCTCTGTCGACCTGTCATATTGAAGGCGACGACCTGCATA
TCCAGCGTAATGTTCAGAAGCTTAAGGACACTGTAAAAAAGCTGGGTGAGAGC
GGTGAGATCAAAGCGATTGGTGAGCTGGACTTGCTGTTCATGTCCTTGCGCAAC
GCCTGCATTCATCACCATCACCACCAC (SEQ ID NO: 5).

The mutants described in the present invention are generated based on human IL-22. The human IL-22 may include a signal peptide or may lack a signal peptide. In the embodiments of the present invention, all test candidates do not contain the signal peptide. The signal peptide comprises a total of 33 amino acid residues, and its amino acid sequence is MAALQKSVSSFLMGTLATSCLLLLALLVQGGAA (SEQ ID NO:7). All conjugation sites or mutation sites referred to in the claims or specification of the present invention are numbered according to the IL-22 sequence without the signal peptide (SEQ ID NO:1). For example, the mutation site D10 pAcF refers to substitution of the D (i.e., Asp) at position 10 of the amino acid sequence shown in SEQ ID NO:1 with pAcF.

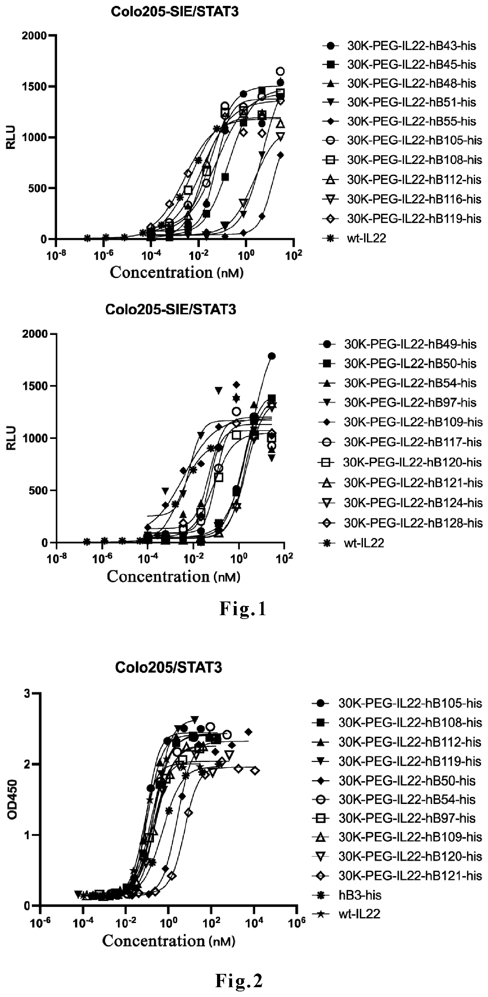

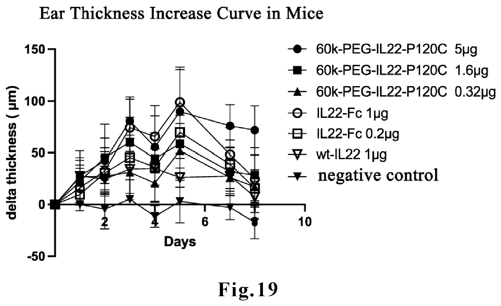

For consistency with the original experimental records, the naming of the mutants adopts the position numbering of the IL-22 protein that includes the signal peptide. Specifically, if the residue position in the amino acid sequence shown in SEQ ID NO:1 is designated as X, then the corresponding amino acid residue position in the protein containing the signal peptide shown in SEQ ID NO:7 is X+33. In the embodiments of the present invention, the mutation sites described in Tables 1-18 and Figures 1-19 are numbered according to the corresponding positions in SEQ ID NO:1, whereas the mutant names are based on the corresponding amino acid positions in the protein containing the signal peptide. For example, the mutation site D10pAcF corresponds to the mutant named IL22-hB43-his. This mutation site is located at position 10 in the protein sequence without the signal peptide, and at position 10+33, i.e., position 43, in the protein sequence containing the signal peptide.

In the examples, the naming convention for PEG-modified mutants is as follows: PEG MW-PEG-IL-22-amino acid substitution. For example, 80k-PEG-IL-22-D109C indicates that one PEG molecule with a molecular weight of 80 kDa is conjugated to the substituted cysteine residue at position 76 of the IL-22 molecule (where 76 refers to the position of the cysteine residue in the protein sequence without the signal peptide; 76+33=109, and therefore the site is designated as 109 in the mutant name).

The numbering of the sites in the mutant names of the present invention is used for identification purposes only and does not indicate that the prepared mutant contains a signal peptide. All test candidates involved in the examples of the present invention do not contain the signal peptide shown in SEQ ID NO:7. All materials used in the present invention are commercially available products and may be purchased from the market. The present invention is further illustrated below in conjunction with the following examples:

### Comparative Example 1

### 1) Cloning, Expression, and Purification of Natural wt IL-22 (SEQ ID NO:1) in Escherichia coli.

Based on the protein sequence of mature human IL-22 (A34-I179; UniProt: Q9GZX6), a cDNA sequence encoding wt IL-22 (SEQ ID NO:4) was designed according to the codon preference of Escherichia coli. An initiation codon (ATG) and an EcoRI restriction site were added to the 5' end of the cDNA, and three stop codons together with a BamHI restriction site were added to the 3' end. The cDNA was synthesized by DNA synthesis and subsequently cloned into the expression vector pBV220KR1. The expression vector containing the corresponding cDNA sequence was transformed into DH5α competent cells (TaKaRa) using the calcium chloride method. Positive clones were selected from kanamycin-resistant plates and verified by sequencing, and the confirmed clone was used as the expression strain for wt IL-22.

The wt IL-22 expression strain was inoculated into TB medium and cultured at 35°C with shaking at 250 rpm until reaching the logarithmic growth phase. The temperature was then increased to 42°C to induce expression for 4 hours. After cultivation, the bacterial cells were harvested by centrifugation at 13,900 g. The bacterial cells were washed and resuspended in a buffer solution containing 50 mM Tris-HCl, 5 mM EDTA, and pH 8.0. The cells were then lysed at 700-1000 bar and the inclusion bodies were harvested by centrifugation at 13900 g.

The inclusion bodies were washed with 50 mM Tris, 5 mM EDTA, 0.5% DOC, 100 mM NaCl, 1 mM DTT, and pH 8.0, and the washing was repeated once. Washed once with 50 mM Tris, 5 mM EDTA, pH 8.0. Inclusion bodies were resuspended in 7M guanidine hydrochloride, 50mM Tris, and pH 8.0, dispersed at high speed, and then stirred at 2-8°C for 4 hours. The protein solution was diluted 20-fold with a refolding buffer containing 0.5 M arginine, 100 mM Tris, 1 mM GSH, and 0.1 mM GSSG (pH 8.0), and allowed to stand at 2-8°C for at least 48 hours. The refolded solution was concentrated and buffer-exchanged by ultrafiltration into the solution of 5% trehalose and 10 mM NaAc, pH 4.5. After filtration, ion-exchange chromatography was performed using CM Sepharose FF (Cytiva). The column was equilibrated with 3-5 column volumes (CV) of 50 mM NaAc buffer (pH 5.0). The protein solution was then loaded onto the column. After sample loading, the column was washed with 50 mM NaAc buffer (pH 5.0), followed by pre-washing with 2.5 CV of the solution of 50 mM NaAc, 250 mM NaCl (pH 5.0). The column was subsequently re-equilibrated with 3-5 CV of 50 mM NaAc buffer (pH 5.0). Elution was performed using elution solution of 50 mM NaAc and 400 mM NaCl (pH 5.0), and the eluate was collected.

### Comparative Example 2: Cloning of Control IL-22 Mutant (hB3-His, SEQ ID NO:2)

With reference to the IL-22 mutant 22-B3 described in WO2021212056 and "The tissue protective functions of interleukin-22 can be decoupled from pro-inflammatory actions through structure-based design (Immunity. 2021;54(4):660-672.e9)", the following amino acid substitutions were introduced into wt IL-22: S12E, Q83A, R91A, and R95A. A 6×His tag was added to the C-terminus.

According to the codon preference of *Escherichia coli*, a cDNA sequence encoding hB3-His (SEQ ID NO:5) was designed. An initiation codon (ATG) and an NdeI restriction site were added to the 5' end of the cDNA, and a stop codon and an EcoRI restriction site were added to the 3' end. The cDNA was synthesized by DNA synthesis and subsequently cloned into the expression vector pET30a(+). The expression vector containing the corresponding cDNA sequence was transformed into BL21 (DE3) competent cells (Invitrogen) using the calcium chloride method. Positive clones were selected from kanamycin-resistant plates and verified by sequencing, and the confirmed clones were used as expression strains for the mutant proteins.

The hB3-His expression strain was inoculated into auto-induction medium containing 0.2% lactose and cultured overnight at 37°C with shaking at 250 rpm to express the mutant protein. The bacterial cells were harvested by centrifugation at 13,900 g. The bacterial cells were washed and resuspended in a buffer solution of 50 mM Tris-HCl, 5 mM EDTA, and pH 8.0. The cells were then lysed at 700-1000 bar and the inclusion bodies were harvested by centrifugation at 13900 g.

The inclusion bodies were washed sequentially with buffer containing 50 mM Tris, 5 mM EDTA, 0.5% DOC, 100 mM NaCl, and 1 mM DTT (pH 8.0), followed by washing with 50 mM Tris and 5 mM EDTA (pH 8.0). The inclusion bodies were then solubilized in the buffer of 7 M guanidine hydrochloride and 50 mM Tris (pH 8.0). The protein solution was diluted 20-fold with a refolding buffer containing 0.5 M arginine, 100 mM Tris, 1 mM GSH, and 0.1 mM GSSG (pH 8.0), and allowed to stand at 2-8°C for at least 48 hours. The refolded solution was concentrated and buffer-exchanged by ultrafiltration into the solution of 5% trehalose and 10 mM NaAc, pH 4.5. After filtration, ion-exchange chromatography was performed using CM Sepharose FF (Cytiva). The column was equilibrated with 3-5 column volumes (CV) of 40 mM NaAc buffer (pH 5.2), and the protein solution was loaded onto the column. After sample loading, the column was washed with 40 mM NaAc buffer (pH 5.2). Gradient elution was performed with 10 CV of the solution of 40 mM NaAc,1 M NaCl (pH 5.2) using a 0-1 M NaCl gradient, and the eluate was collected. The collected eluate was ultrafiltered using an ultrafiltration tube and then loaded onto a Superdex 75pg (120ml, Cytiva) for chromatography to collect the target protein peak.

### Comparative Example 3: Cloning, Expression, and Purification of IL-22-Fc (Code Name: RG7880, SEQ ID NO: 3).

The plasmid of RG7880 (gene synthesized by Beijing Tsingke Biotechnology Co., Ltd.) was transfected into CHO-K1 cells. At 24 h after transfection, 10 µg/mL MSX was added for selection under pressure. After the cell density and viability recovered, the cells were seeded for fed-batch expression. The centrifuged supernatant obtained after completion of expression was sequentially purified by protein A, Capto adhere, and Phenyl HP hydrophobic chromatography, and the antibody concentration was quantified by the BCA method.

### Example 1 Cloning of Non-Natural Amino Acid IL-22 Mutant (6*His) in Escherichia Coli

According to the mutation sites shown in Table 1, the parental amino acid codon at each site of wt IL-22 (SEQ ID NO:1) was replaced with the amber codon TAG (so that the amino acid at the corresponding site was mutated to p-acetyl-L-phenylalanine). In order to add a 6*his tag at the C-terminus, CATCACCACCACCACCAC (SEQ ID NO:6) was added to the C-terminus of the nucleic acid sequence (SEQ ID NO:4) optimized based on the codon preference of Escherichia coli. A cDNA encoding the IL22 mutant corresponding to each codon mutation shown in Table 1 was designed. A start codon ATG and an NdeI restriction site were added at the 5' end of the cDNA, and a stop codon and an XhoI restriction site were added at the 3' end. The cDNA was prepared by DNA synthesis, and then cloned into the expression vector pET30a(+). Using the calcium chloride method, competent BL21 (DE3) cells (Invitrogen) were co-transformed with the expression vector pET30a(+) containing the corresponding cDNA sequence and the auxiliary plasmid pEVOL-pAcF capable of specifically incorporating p-acetyl-L-phenylalanine (pAcF). Positive clones were selected from plates containing kanamycin and chloramphenicol, and after sequencing verification, were used as the expression strains of each mutant protein.

**Table 1 List of IL22 Mutants**

| Category | Mutant name | Mutation site |
|---|---|---|
| | IL22-hB43-his | D10 pAcF |
| | IL22-hB45-his | S12 pAcF |
| | IL22-hB48-his | Q15 pAcF |
| | IL22-hB49-his | Q16 pAcF |
| | IL22-hB50-his | P17 pAcF |
| | IL22-hB51-his | Y18 pAcF |
| | IL22-hB54-his | N21 pAcF |
| | IL22-hB55-his | R22 pAcF |
| | IL22-hB97-his | N64 pAcF |
| Non-natural amino acid mutation | IL22-hB105-his | F72 pAcF |
| | IL22-hB108-his | S75 pAcF |
| | IL22-hB109-his | D76pAcF |
| | IL22-hB112-his | Q79 pAcF |
| | IL22-hB116-his | Q83 pAcF |
| | IL22-hB117-his | E84 pAcF |
| | IL22-hB119-his | V86 pAcF |
| | IL22-hB120-his | P87 pAcF |
| | IL22-hB121-his | F88 pAcF |
| | IL22-hB124-his | R91 pAcF |
| | IL22-hB128-his | R95 pAcF |

The expression strains of each non-natural amino acid mutant protein were inoculated into auto-induction medium containing 1 mM p-acetyl-L-phenylalanine, 0.02% arabinose, and 0.2% lactose. The cultures were incubated at 37°C with shaking at 250 rpm overnight to express the respective mutant proteins. The bacterial cultures were centrifuged at 13,900 g to harvest the bacterial cells. The bacterial cells were washed and resuspended in a buffer solution of 50 mM Tris-HCl, 5 mM EDTA, and pH 8.0. The cells were then lysed at 700-1000 bar and the inclusion bodies were harvested by centrifugation at 13900 g.

The inclusion bodies were washed with a washing buffer containing 0.5% DOC, and the precipitate was collected by centrifugation. The crude inclusion bodies were dissolved in a buffer solution containing 50 mM MES, 8 M urea, 10 mM EDTA, and 0.1 mM DTT (pH 5.5), followed by centrifugation to collect the supernatant. The supernatant was added to pre-cooled refolding buffer (100 mM Tris, 2 mM EDTA, 0.5 M L-Arg, 1 mM GSH, 0.1 mM GSSG, pH 8.0) at a ratio of 1:20 (v/v), and allowed to stand at 2-8°C for approximately 72 h. The refolded solution was concentrated and buffer-exchanged by ultrafiltration into buffer solution containing 10 mM NaAc, 5% trehalose (pH 4.5), followed by filtration and purification by CM Sepharose FF (Cytiva) ion exchange chromatography. The buffers solution used for CM Sepharose FF ion exchange chromatography were as follows: Buffer A: 20 mM MES, pH 6.0; Buffer B: 20 mM MES, 1 M NaCl, pH 6.0. The chromatography column was first equilibrated with 3 to 5 column volumes of buffer A, and then the refolded solution was loaded onto the chromatography column. After loading, the chromatography column was rinsed with buffer A, and then gradient eluted with 10 column volumes of 0 to 100% buffer B to collect the purified samples.

### Example 2 Non-tagged Non-natural Amino Acid Mutants of IL22

According to the mutation sites shown in Table 2, the parental amino acid codon at each site of wt IL-22 was replaced with the amber codon TAG (so that the amino acid at the corresponding site was mutated to p-acetyl-L-phenylalanine). According to the codon preference of *Escherichia coli*, a cDNA encoding the corresponding IL22 mutant for each codon mutation shown in Table 2 was designed, and a start codon ATG and an NdeI restriction site were added at the 5' end of the cDNA, and three stop codons and an XhoI restriction site were added at the 3' end. The cDNA was prepared by DNA synthesis, and then cloned into the expression vector pET30a(+). Using the calcium chloride method, competent BL21 (DE3) cells (Invitrogen) were co-transformed with the expression vector pET30a(+) containing the corresponding cDNA sequence and the auxiliary plasmid pEVOL-pAcF capable of specifically incorporating p-acetyl-L-phenylalanine (pAcF). Positive clones were selected from plates containing kanamycin and chloramphenicol, and after sequencing verification, were used as the expression strains of each mutant protein.

**Table 2. List of Non-Natural Amino Acid Mutants of IL22**

| Mutant name | Mutation site |
|---|---|
| IL22-hB50 | P17 pAcF |
| IL22-hB120 | P87 pAcF |
| IL22-hB121 | F88 pAcF |

The expression strains of each IL22-pAcF mutant protein were inoculated into auto-induction medium containing 1 mM p-acetyl-L-phenylalanine, 0.02% arabinose, and 0.2% lactose. The cultures were incubated at 37°C with shaking at 250 rpm overnight to express the respective mutant proteins. The bacterial cultures were centrifuged at 13,900 g to harvest the bacterial cells. The bacterial cells were washed and resuspended in a buffer solution containing 50 mM Tris-HCl, 5 mM EDTA, and pH 8.0. The cells were then lysed at 700-1000 bar and the inclusion bodies were harvested by centrifugation at 13900 g.

The inclusion bodies were washed sequentially with a buffer solution containing 2 M urea, 2% Triton X-100, 50 mM Tris, 5 mM EDTA, pH 8.0, and with a buffer solution containing 50 mM Tris, 5 mM EDTA, pH 8.0. The inclusion bodies were dissolved in 7 M guanidine hydrochloride, 50 mM Tris, pH 8.0. The protein solution was diluted 20-fold with a refolding buffer containing 0.5 M arginine, 100 mM Tris, 1 mM GSH, 0.1 mM GSSG, pH 8.0, and allowed to stand at 2-8°C for 48 h. The refolded solution was concentrated and buffer-exchanged by ultrafiltration into buffer solution containing 10 mM NaAc, 5% trehalose (pH 4.5), followed by filtration and purification by Capto SP ImpRes (Cytiva) ion exchange chromatography. The column was equilibrated with 3-5 column volumes (CV) of 20 mM NaAc buffer (pH 5.5), and the protein solution was loaded onto the column. After sample loading, the column was washed with 20 mM NaAc buffer (pH 5.5). Gradient elution was performed using 10 CV of 20 mM NaAc,1 M NaCl (pH 5.5) with a 0-1 M NaCl gradient, and the eluate was collected.

### Example 3: Polyethylene Glycol Modification and Purification of IL22 Non-Natural Amino Acid Mutants.

The purified proteins obtained in Example 1 and Example 2 were subjected to ultrafiltration for buffer exchange into 10 mM NaAc, 5% trehalose, pH 4.5, to a final concentration of 5-10 mg/mL. Ammonium chloride was added at a molar ratio of 1:125 (IL22:ammonium chloride); acetyl hydrazine was added at a molar ratio of 1:30 (IL22:acetyl hydrazine); PEG-hydroxylamine (e.g., Mw 30 kDa, 40 kDa, Xiamen Sinopeg) was added at a molar ratio of 1:5 (IL22:PEG). The reaction was carried out at 2-8°C for approximately 48-60 h to conjugate PEG to the mutation site of each mutant. The reaction solution was diluted 10-fold with purified water, and the pH was adjusted to 5.5, followed by purification by Macro Cap SP (Cytiva) ion exchange chromatography. After equilibration with 3-5 column volumes of 40 mM NaAc, pH 5.5 buffer, the protein solution was loaded onto the chromatography column. After loading was completed, the column was washed with 40 mM NaAc buffer, pH 5.5, and gradient elution was performed with the buffer of 0-0.5 M NaCl, 40 mM NaAc, pH 5.5, and the modified proteins were collected.

### Example 4 Cloning, Expression, and Purification of IL22 Cysteine Mutants in Escherichia coli

According to the mutation sites shown in Table 3 (with SEQ ID NO:1 as the wild type), the parental amino acid at each site was replaced with cysteine (abbreviated as Cys). According to the codon preference of *Escherichia coli*, a cDNA encoding the corresponding IL22 mutant for each codon mutation shown in Table 3 was designed, and a start codon ATG and an EcoRI restriction site were added at the 5' end of the cDNA, and three stop codons and a BamHI restriction site were added at the 3' end. The cDNA was prepared by DNA synthesis, and then cloned into the expression vector pBV220KR1. Using the calcium chloride method, the expression vector containing the corresponding cDNA sequence was transformed into DH5α competent cells (TaKaRa). Positive clones were selected from kanamycin-resistant plates, and after sequencing verification, were used as the expression strains of each mutant protein.

**Table 3 List of IL22 Cysteine Mutants**

| Category | Mutant name | Mutation site |
|---|---|---|
| | IL22-P50C | 17 |
| Cysteine mutation | IL22-D109C | 76 |
| | IL22-P120C | 87 |
| | IL22-F121C | 88 |

The expression strain of each IL22 cysteine mutant protein was inoculated into TB medium and cultured at 35°C with shaking at 250 rpm until reaching the logarithmic growth phase. The temperature was then increased to 42°C to induce expression for 4 hours. After cultivation, the bacterial cells were harvested by centrifugation at 13,900 g. The bacterial cells were washed and resuspended in a buffer solution containing 50 mM Tris-HCl, 5 mM EDTA, and pH 8.0. The cells were then lysed at 700-1000 bar and the inclusion bodies were harvested by centrifugation at 13900 g.

The inclusion bodies were washed sequentially with a buffer solution containing 50 mM Tris, 5 mM EDTA, 2% Triton X-100, pH 8.0, and with a buffer solution containing 50 mM Tris, 5 mM EDTA, pH 8.0. The inclusion bodies were dissolved in 8 M urea, 50 mM MES, 0.1 mM DTT, and 10 mM EDTA, pH 5.5. The protein solution was diluted 20-fold with a refolding buffer containing 0.5 M arginine, 100 mM Tris, 1 mM GSH, 0.1 mM GSSG, pH 8.0, and allowed to stand at 2-8°C for 48 h. The refolded solution was concentrated and buffer-exchanged by ultrafiltration into buffer solution containing 10 mM NaAc, 5% trehalose (pH 4.5), followed by filtration and purification by CM Sepharose FF (Cytiva) ion exchange chromatography. The column was equilibrated with 3-5 column volumes (CV) of 40 mM NaAc buffer (pH 5.1), and the protein solution was loaded onto the column. After sample loading, the column was washed with 40 mM NaAc buffer (pH 5.1). Gradient elution was performed using 10 CV of 40 mM NaAc,1 M NaCl (pH 5.1) with 0-100%, and the eluate was collected.

### Example 5 PEGylation and Purification of IL22 Cysteine Mutants

Each of IL22 cysteine mutants obtained in Example 4 was modified with polyethylene glycol. The PEG used in this example contains a maleimide group, which can react specifically with the cysteine residue at a specific mutation site of the mutant, thereby allowing the PEG to site-specifically modify the corresponding IL-22 mutant.

The purified IL-22 cysteine mutant protein was adjusted to pH 6.0. PEG-maleimide (e.g., Mw 40 kDa, 60 kDa, 80 kDa PEG, Xiamen Sinopeg) was weighed at a molar ratio of IL-22 mutant to PEG of 1:5. The PEG powder was dissolved in purified water to a final concentration of 100 mg/mL and then added to the protein solution. After mixing uniformly, the reaction was carried out overnight at 2-8°C.

The reaction solution was diluted 10-fold with purified water, and the pH was adjusted to 5.1, followed by purification by Macro Cap SP (Cytiva) ion exchange chromatography. After equilibration with 3-5 column volumes of 40 mM NaAc buffer, pH 5.1, the protein solution was loaded onto the chromatography column. After loading was completed, the column was washed with 40 mM NaAc buffer, pH 5.1, and gradient elution was performed with 0-0.5 M NaCl in 40 mM NaAc buffer, pH 5.1, and the modified proteins were collected.

**Table 4 List of Information on PEG-IL22 Cysteine Mutant test candidate**

| Mutant name | Conjugation site | PEG molecular weight |
|---|---|---|
| 40K-PEG-IL22-P50C | 17 | 40kD |
| 60K-PEG-IL22-P50C | 17 | 60kD |
| 80K-PEG-IL22-P50C | 17 | 80kD |
| 40K-PEG-IL22-D109C | 76 | 40kD |
| 60K-PEG-IL22-D109C | 76 | 60kD |
| 80K-PEG-IL22-D109C | 76 | 80kD |
| 40K-PEG-IL22-P120C | 87 | 40kD |
| 60K-PEG-IL22-P120C | 87 | 60kD |
| 80K-PEG-IL22-P120C | 87 | 80kD |
| 40K-PEG-IL22-F121C | 88 | 40kD |
| 60K-PEG-IL22-F121C | 88 | 60kD |
| 80K-PEG-IL22-F121C | 88 | 80kD |

### Effect Verification

### 1. Evaluation of STAT3 Activation Activity of PEG-IL22 Mutants Modified at Different Sites Using a Reporter Gene Assay

COLO205-SIE cells were constructed to evaluate the STAT3 activation effects of the test candidate.

Specific methods: COLO205 cells were cultured in culture dishes with RPMI1640 + 10% FBS until a sufficient quantity was reached. COLO205 cells were transfected with the pGL4.47[*luc2P*/SIE/Hygro](Promega, E4041) plasmid using the NEON^{™} electroporation transfection system (Invitrogen) to obtain COLO205/SIE cells. The cells were resuspended in RPMI1640 + 10% FBS to a concentration of 1× 10⁶ cells/ml. 50 µl/well was added to each well of a 96-well plate and incubated overnight at 37°C with 5% CO₂ (approximately 16 h). IL-22 samples were serially diluted and added to each well (50 µl/well). After incubation at 37°C with 5% CO₂ for 6 h, 100 µl/well of Bio-Lite Luciferase Assay System (Vazyme, Cat:DD1201-02) was added, and chemiluminescence values were detected. The logarithmic values of the final concentrations of IL-22 samples were plotted as the X-axis, and the corresponding measured values for each well were plotted as the Y-axis. The regression curves for STAT3 phosphorylation reporter gene activity in COLO205/SIE cells stimulated by IL22 were obtained by four-parameter regression fitting in GraphPad Prism 9 software.

Based on the STAT3 reporter gene detection results of COLO205-SIE cells, molecules with better STAT3 phosphorylation activity (lower EC₅₀) were selected for subsequent STAT3/STAT1 preference studies.

**Table 5. EC₅₀ Values for STAT3 Activation by PEG-IL22 Mutants Determined by Reporter Gene Assay**

| Test No. | Mutant name | EC₅₀ for STAT3 Activation (nM) | EC₅₀ fold change of the test candidate relative to wt IL-22 (lower is better) |
|---|---|---|---|
| Assay1 | 30K-PEG-IL22-hB43-his | 0.05555 | 11.1 |
| | 30K-PEG-IL22-hB45-his | 0.191 | 38.1 |
| | 30K-PEG-IL22-hB48-his | 0.05477 | 10.9 |
| | 30K-PEG-IL22-hB51-his | 5.083 | 1013.3 |
| | 30K-PEG-IL22-hB55-his | 13.13 | 2609.8 |
| | 30K-PEG-IL22-hB105-his | 0.03596 | 7.3 |
| | 30K-PEG-IL22-hB108-his | 0.01473 | 2.9 |
| | 30K-PEG-IL22-hB112-his | 0.01392 | 2.8 |
| | 30K-PEG-IL22-hB116-his | 2.148 | 427.4 |
| | 30K-PEG-IL22-hB119-his | 0.00265 | 0.5 |
| | wt IL-22 | 0.00529 | 1.0 |
| Assay2 | 30K-PEG-IL22-hB49-his | 3.314 | 1149.9 |
| | 30K-PEG-IL22-hB50-his | 1.452 | 505.1 |
| | 30K-PEG-IL22-hB54-his | 0.05186 | 18.3 |
| | 30K-PEG-IL22-hB97-his | 0.00699 | 2.5 |
| | 30K-PEG-IL22-hB109-his | 0.00375 | 1.3 |
| | 30K-PEG-IL22-hB117-his | 0.0987 | 34.3 |
| | 30K-PEG-IL22-hB120-his | 0.07733 | 26.9 |
| | 30K-PEG-IL22-hB121-his | 1.849 | 641.1 |
| | 30K-PEG-IL22-hB124-his | 2.144 | 743.1 |
| | 30K-PEG-IL22-hB128-his | 0.05299 | 18.4 |
| | wt IL-22 | 0.00303 | 1.0 |

The activity of the PEGylated IL-22 non-natural amino acid mutant prepared in Example 3 in activating the STAT3 reporter gene at different concentrations is shown in Figure 1.

### 2. COLO205 STAT3/STAT1 Preference

ELISA method was used to screen molecules with potential preference advantages in p-STAT1/p-STAT3 activation in COLO205 cells.

COLO205 cells were cultured in RPMI 1640 medium supplemented with 10% FBS in culture dishes until sufficient cell numbers were obtained. An appropriate amount of cell suspension was collected and centrifuged, washed twice with PBS, and resuspended to a density of 8 × 10⁵ cells/mL. 50 µL per well was added to a 96-well plate and incubated at 37°C with 5% CO₂ for 6 hours. IL-22 samples were serially diluted and added at 50 µL per well. After incubation at 37°C with 5% CO₂ for 0.5 hours, the 96-well plate was placed on ice for 5-10 minutes. The cell lysis buffer provided in the kit was prepared as a 2× solution, and 100 µL per well was added to the 96-well plate. The 96-well plate was kept at 4°C or on ice for 5-10 minutes, then transferred to -80°C and stored for not less than 30 minutes, followed by thawing at room temperature.

Microplate strips provided by the STAT1 kit (PathScan^{®} Phospho-Stat1 (Tyr701) Sandwich ELISA Kit, CST, Cat: 73582) and by the STAT3 kit (PathScan^{®} Phospho-Stat3 (Tyr705) Sandwich ELISA Antibody Pair, CST, Cat: 7300) were allowed to reach room temperature before use, and the required number of wells was detached. The wells were placed in a strip holder. Unused wells were resealed and immediately stored at 4°C.

90 µL of each cell lysate was added to the appropriate wells of the microplate provided by STAT1 or STAT3 kit. The microplate was sealed with sealing film, pressed firmly on top of the microwells and incubated at 37°C for 2 hours or overnight at 4°C.

The contents were discarded, and the wells were washed four times with 1× washing buffer (200 µL per well each time). After each wash, the plate was tapped firmly on clean absorbent paper to remove residual liquid in each of wells, but the wells were not allowed to dry completely. 100 µL of reconstituted detection antibody was added to each well. The plate was sealed with sealing film and incubated at 37°C for 1 hour, repeating the washing procedure. Next, 100 µL of reconstituted HRP-labeled secondary antibody was added to each well. The plate was sealed with tape and incubated at 37°C for 30 minutes, repeating the washing procedure. Then, 100 µL of TMB substrate was added to each well. The plate was sealed with sealing film and incubated at 37°C for 10 minutes or at 25°C for 30 minutes. Subsequently, 100 µL of STOP solution was added to each well and gently shaken for several seconds. The absorbance was read at 450 nm within 30 minutes after addition of the STOP solution. The logarithmic values of the final concentrations of IL-22 samples were plotted as the X-axis, and the corresponding measured values for each well were plotted as the Y-axis. Regression curves for the phosphorylation activities of STAT1 and STAT3 in COLO205 cells stimulated by IL22 were obtained by four-parameter regression fitting of GraphPad Prism 9 software.

STAT3/STAT1 preference was evaluated using the ratio of the EC₅₀ values for activated inducing STAT3 and STAT1 phosphorylation. The COLO205 STAT3/STAT1 preference results showed that among all test candidates, 30K-PEG-IL22-hB50 and 30K-PEG-IL22-hB120 exhibited better preference for STAT3/STAT1 activation compared with wt IL-22. Although the EC₅₀ preference of 30K-PEG-IL22-hB121 was not as good as that of the above two molecules, its STAT1 phosphorylation activation Emax was lower, indicating a lower activation capacity even at high concentrations.

**Table 6. EC₅₀ values for STAT1/STAT3 activation of PEG-IL22 mutants determined by ELISA**

| Mutant name (test candidate) | STAT3 Activation | | STAT1 Activation | | STAT1/ST AT3 Ratio (higher is better) |
|---|---|---|---|---|---|
| | EC₅₀ (nM) | Fold change relative to wt IL-22 | EC₅₀ (nM) | Fold change relative to wt IL-22 | |
| 30K-PEG-IL22-hB 105-his | 0.0955 6 | 1.44 | 2.391 | 2.55 | 1.77 |
| 30K-PEG-IL22-hB 108-his | 0.1714 | 2.59 | 2.596 | 2.77 | 1.07 |
| 30K-PEG-IL22-hB 112-his | 0.1089 | 1.64 | 3.396 | 3.62 | 2.20 |
| 30K-PEG-IL22-hB 119-his | 0.2377 | 3.59 | 1.128 | 1.20 | 0.34 |
| 30K-PEG-IL22-hB 50-his | 2.581 | 38.94 | 274.8 | 292.71 | 7.52 |
| 30K-PEG-IL22-hB 54-his | 0.2013 | 3.04 | 4.856 | 5.17 | 1.70 |
| 30K-PEG-IL22-hB 97-his | 0.2151 | 3.25 | 5.701 | 5.89 | 1.81 |
| 30K-PEG-IL22-hB 109-his | 0.2689 | 4.06 | 5.118 | 5.45 | 1.34 |
| 30K-PEG-IL22-hB 120-his | 0.2101 | 3.17 | 117.8 | 125.48 | 39.58 |
| 30K-PEG-IL22-hB 121-his | 5.691 | 85.86 | 110.2 | 117.38 | 1.37 |
| hB3-his | 0.5252 | 7.92 | 17.39 | 18.52 | 2.34 |
| wt IL-22 | 0.0662 8 | 1 | 0.9388 | 1 | 1 |

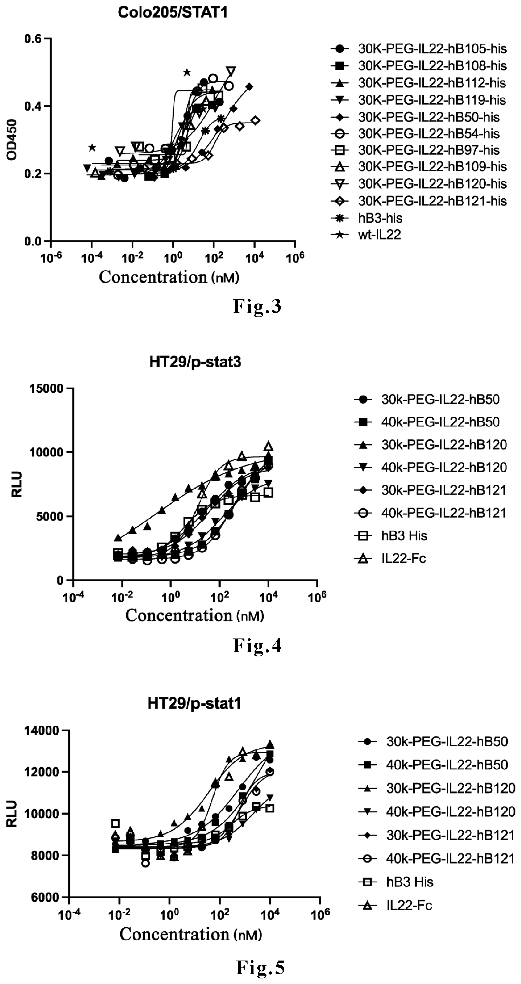

In addition, the activity of PEGylated IL-22 non-natural amino acid mutants in activating STAT3 phosphorylation in COLO205 cells at different concentrations, as determined by ELISA, is shown in Figure 2. The activity of PEGylated IL-22 non-natural amino acid mutants in activating STAT1 phosphorylation in COLO205 cells at different concentrations, as determined by ELISA, is shown in Figure 3.

### 3. Effect of Different Molecular Weights of PEG Modification on IL-22 Receptor Preference

The tag-free IL-22 non-natural amino acid mutant prepared in Example 2 was used. IL-22 mutant products modified with PEGs of different molecular weights were prepared according to the method described in Example 3. The details are shown in the table below:

**Table 7. Information of PEG-IL22 non-natural amino acid mutant test candidate**

| Mutant name (test candidate) | Conjugation site | PEG molecular weight |
|---|---|---|
| 30K-PEG-IL22-hB50 | 17 | 30kD |
| 40K-PEG-IL22-hB50 | 17 | 40kD |
| 30K-PEG-IL22-hB120 | 87 | 30kD |
| 40K-PEG-IL22-hB120 | 87 | 40kD |
| 30K-PEG-IL22-hB121 | 88 | 30kD |
| 40K-PEG-IL22-hB121 | 88 | 40kD |

HT-29 adherent cells (a human colon carcinoma cell line) were selected, and FACS was used to detect the preference of PEGylated IL-22 for activation of pSTAT1 and pSTAT3 phosphorylation. hB3-His and IL-22-Fc were used as control molecules.

On the day prior to the experiment, cells were seeded into a 96-well plate at 2 × 10⁵ cells per well and incubated overnight at 37°C with 5% CO₂ to allow adherence. Different test candidates were serially diluted in McCoy's medium containing 10% FBS, and 100 µL per well was added. The cells were incubated at 37°C with 5% CO₂ for 30 minutes. Cells were washed once with 100 µL PBS per well. After aspiration, 25 µL per well of trypsin (Gibco, 25200-560) was added and incubated at 37°C for approximately 5 minutes to generate suspended cells. The plate was gently tapped to disperse the cells. Then, 80 µL per well of McCoy's medium containing 10% FBS was added to neutralize, and the cells were pipetted to obtain a single-cell suspension. 100 µL from each well was transferred to a V-bottom plate and centrifuged. Cells were resuspended in 100 µL per well of pre-warmed 1× fixation buffer (BD Biosciences, 558049) or 4% paraformaldehyde (Sigma-Aldrich, 158127-5G) and incubated at 37°C for 15-20 minutes for fixation. After centrifugation and removal of the fixation solution, the cells were first gently dispersed using the residual liquid. Then, 80 µL per well of -20°C pre-cooled permeabilization buffer (BD Biosciences, 558050) was added to fully resuspend the cells. The cells were permeabilized on ice for 0.5-1 hour, with gentle tapping 2-3 times during the process to prevent cell aggregation. After thoroughly washing with PBS and centrifugation, flow cytometry antibodies (anti-STAT1 pY701, BD Biosciences 612596; anti-STAT3 pY705, BD Biosciences 557815) were added and incubated in the dark for 30-60 minutes. After washing again, the samples were analyzed by flow cytometry to determine the MFI values corresponding to FITC and APC for each sample.

The logarithmic values of the final concentrations of each sample were plotted as the X-axis, and the corresponding MFI values were plotted as the Y-axis. Regression curves for the phosphorylation activities of STAT1/STAT3 in HT-29 cells stimulated by IL22-related test candidates were obtained by four-parameter regression fitting of GraphPad Prism 9 software.

The activity of PEGylated IL-22 non-natural amino acid mutants in activating STAT3 phosphorylation in HT29 cells at different concentrations, as determined by FACS, is shown in Figure 4. The activity of PEGylated IL-22 non-natural amino acid mutants in activating STAT1 phosphorylation in HT29 cells at different concentrations, as determined by FACS, is shown in Figure 5.

Compared with the control molecules IL-22-Fc and hB3-His, all 40k-PEG-modified IL-22 mutants significantly reduced STAT3 phosphorylation activity and were far lower than IL-22-Fc. All 30k-PEG-modified IL-22 mutants retained relatively good STAT3 phosphorylation activity and exhibited enhanced preference compared with IL-22-Fc. Compared with IL-22-Fc, 30k-PEG-IL22-hB120 showed approximately a 20-fold increase in STAT3 phosphorylation activity under conditions where STAT1 phosphorylation activity was comparable. For 30k-PEG-IL22-hB50 and 30k-PEG-IL22-hB121, under conditions where STAT3 activity was comparable, STAT1 phosphorylation activity was significantly reduced.

**Table 8. Preference of PEG-IL22 non-natural amino acid mutants for STAT1/STAT3 phosphorylation activation determined by FACS**

| Mutant name (test candidate) | EC₅₀ for STAT3 Activation (nM) | EC₅₀ for STAT1 Activation (nM) | STAT1/STAT3 EC₅₀ ratio (higher is better) |
|---|---|---|---|
| 30K-PEG-IL22-hB50 | 24.94 | 581.3 | 23.3 |
| 40K-PEG-IL22-hB50 | 532.3 | 4357 | 8.2 |
| 30K-PEG-IL22-hB120 | 0.2443 | 35.53 | 145.4 |
| 40K-PEG-IL22-hB120 | 120.4 | 2112 | 17.5 |
| 30K-PEG-IL22-hB121 | 39.62 | 588.7 | 14.9 |
| 40K-PEG-IL22-hB121 | 300.3 | 658.6 | 2.2 |
| HB3-his | 4.126 | 278.6 | 67.5 |
| IL-22-Fc | 12.89 | 49.91 | 3.9 |

### 4. Determination of pSTAT1 and pSTAT3 Activation Preference by FACS

According to the methods described in the preceding three sections, FACS was used to detect the preference of PEGylated IL-22 for receptor activation. hB3-His and IL-22-Fc were used as control molecules.

**Table 9. Preference of PEG-IL22 cysteine mutants for STAT1/STAT3 phosphorylation activation determined by FACS**

| Mutant name (test candidate) | STAT3 Activation EC₅₀ (nM) | STAT1 Activation EC₅₀ (nM) | STAT1/STAT3 EC₅₀ ratio (higher is better) |
|---|---|---|---|
| 40K-PEG-IL22-P50C | 47.49 | 369.0 | 7.8 |
| 60K-PEG-IL22-P50C | 55.47 | 134.1 | 2.4 |
| 80K-PEG-IL22-P50C | 18.9 | 62.34 | 3.3 |
| 40K-PEG-IL22-D109C | 0.3483 | 10.91 | 31.3 |
| 60K-PEG-IL22-D109C | 0.5769 | 9.411 | 16.3 |
| 80K-PEG-IL22-D109C | 0.984 | 6.996 | 7.1 |
| 40K-PEG-IL22-P120C | 6.048 | 55.3 | 9.1 |
| 60K-PEG-IL22-P120C | 11.37 | 86.22 | 7.6 |
| 80K-PEG-IL22-P120C | 19.81 | 574.2 | 29.0 |
| hB3-his | 2.471 | 22.96 | 9.3 |
| IL-22-Fc | 3.904 | 12.62 | 3.2 |

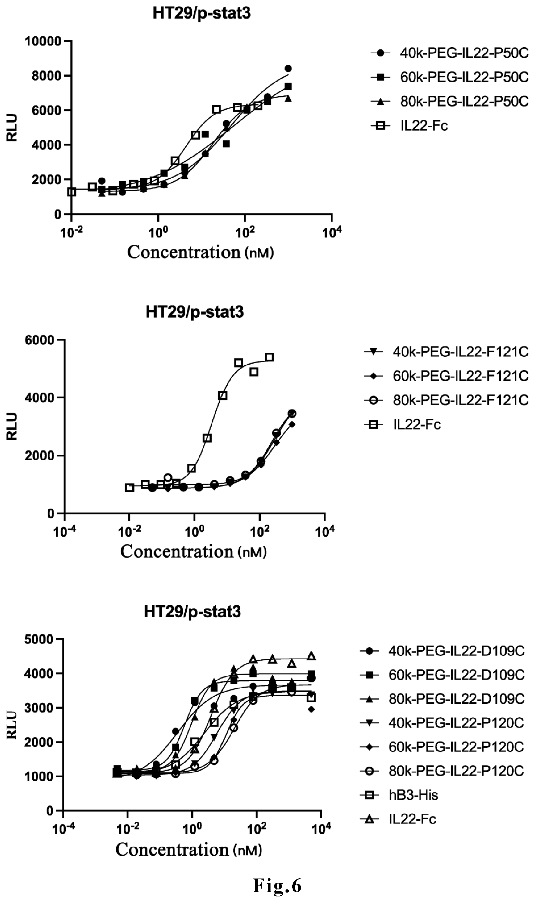

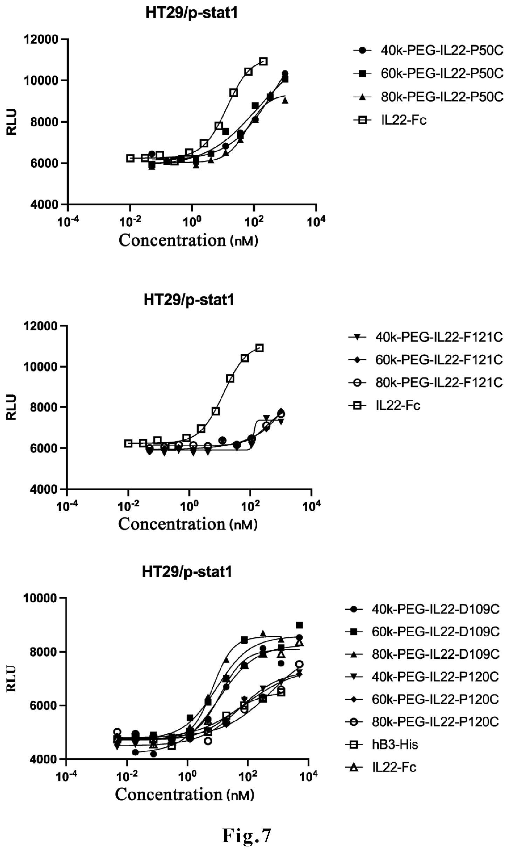

The activity of PEGylated IL-22 cysteine mutants in activating STAT3 phosphorylation in HT29 cells at different concentrations, as determined by FACS, is shown in Figure 6. The activity of PEGylated IL-22 cysteine mutants in activating STAT1 phosphorylation in HT29 cells at different concentrations, as determined by FACS, is shown in Figure 7.

Among the site-specific cysteine-PEGylated IL-22 molecules, both molecules PEG-IL22-D109C and PEG-IL22-P120C exhibited a preference advantage compared with IL-22-Fc. The EC₅₀ ratio of STAT1/STAT3 activation in HT29 cells induced by 40K/60K-PEG-IL22-D109C was significantly higher than that of IL-22-Fc and the control molecule hB3-His. PEG-IL22-P120C not only demonstrated an advantageous EC₅₀ ratio, but also showed a lower Emax in the STAT1 activity curve compared with IL-22-Fc, indicating weaker STAT1 activation at high concentrations.

### 5. Tissue Activation Preference

Human immortalized keratinocyte HaCaT cells were recovered and cultured in MEM/EBSS medium (Cytiva, SH30024.01) supplemented with 15% FBS and 1% P/S (Cytiva, SV30010). When cell confluence reached 70%-80%, cells were digested with 0.25% Trypsin-EDTA (Gibco, 25200-056), terminated with complete medium, and resuspended to 1.25 × 10⁶ cells/mL. After mixed thoroughly, a total of 120 µL per well cells was seeded into a 96-well plate (Corning, 3599) at a density of 1.5 × 10⁵ cells per well and incubated overnight at 37°C with 5% CO₂.

wt IL-22 was prepared at an initial working concentration of 1000 nM, and the remaining samples were prepared at 10000 nM as the starting concentration. Except for 60K-PEG-IL22-D109C, which was serially diluted 5-fold in complete medium, all other samples were serially diluted 4-fold in complete medium. After overnight culture, the medium was removed, and 120 µL per well of each gradient-diluted sample was added to the cells. Cells were stimulated for 30 minutes at 37°C with 5% CO₂. After stimulation, samples were removed and cells were washed once with pre-warmed (37°C) 1× PBS (HyClone, SH30256.01). After aspiration, 25 µL per well of 0.25% Trypsin-EDTA was added for digestion, followed by addition of 75 µL per well of complete medium to terminate the reaction. Cells were pipetted to obtain a single-cell suspension. 80 µL of the mixed single-cell suspension was transferred to a 96-well V-bottom plate (NEST, 701201) containing 80 µL per well of 4% paraformaldehyde (Sigma-Aldrich, 158127-5G), mixed thoroughly, and placed at room temperature for 20 minutes. After centrifugation at 1800 rpm for 5 minutes and removal of the supernatant, 100 µL per well of pre-cooled methanol (Merck, 11.06007.4008) was added for permeabilization. Cells were incubated at 4°C for 1 hour, centrifuged to remove the supernatant, washed once with 1× PBS, and stained with the prepared STAT3 antibody (Alexa Fluor 647 mouse anti-Stat3 (pY705), BD, 557815). After staining at room temperature in the dark for 1 hour, cells were washed twice with 1× PBS and analyzed by flow cytometry to determine the mean fluorescence intensity (MFI).

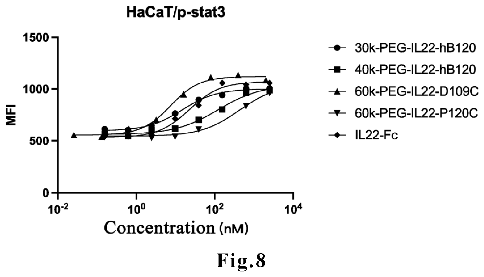

Tissue activation preference of different test candidates was evaluated by comparing their ability to activate STAT3 in intestinal-derived HT29 cells and skin-derived HaCaT cells. The results showed that, compared with IL-22-Fc, 30K-PEG-IL22-hB120, 60K-PEG-IL22-D109C, and 60K-PEG-IL22-P120C demonstrated superior tissue preference, preferentially activating intestinal-derived HT29 cells while showing relatively weaker activation of skin-derived HaCaT cells. This suggests that, in vivo, these molecules may preferentially act on intestinal target organs and are less likely to activate skin cells and cause adverse effects. The activity of PEGylated IL-22 mutants in activating STAT3 phosphorylation in HaCaT cells at different concentrations as determined by FACS is shown in Figure 8.

**Table 10. Tissue preference of PEG-IL22 mutants for STAT3 phosphorylation in human intestinal and human skin cell lines determined by FACS**

| Mutant name (test candidate) | p-STAT3 EC₅₀ (nM) | | HaCaT: HT29 ratio (higher is better) |
|---|---|---|---|
| | HT29 | HaCaT | |
| 30k-PEG-IL22-hB120 | 0.2443 | 14.83 | 60.7 |
| 40k-PEG-IL22-hB120 | 120.4 | 125.4 | 1.0 |
| 60K-PEG-IL22-D109C | 0.5769 | 7.436 | 12.9 |
| 60K-PEG-IL22-P120C | 11.37 | 428.3 | 37.7 |
| IL-22-Fc | 3.904 | 22.66 | 5.8 |

### 6. Mouse PD (Reg3β)

The ELISA method was used to determine the baseline levels and post-dose changes of the biomarker Reg3β in plasma samples before and after administration of different test candidates.

For the preferable test candidates, one mouse per dose group was subcutaneously injected at 0.5, 1, or 3 mg/kg. The control molecules were IL-22-Fc, administered subcutaneously at 0.3 and 1 mg/kg, and wt IL-22, administered intraperitoneally at the corresponding doses.

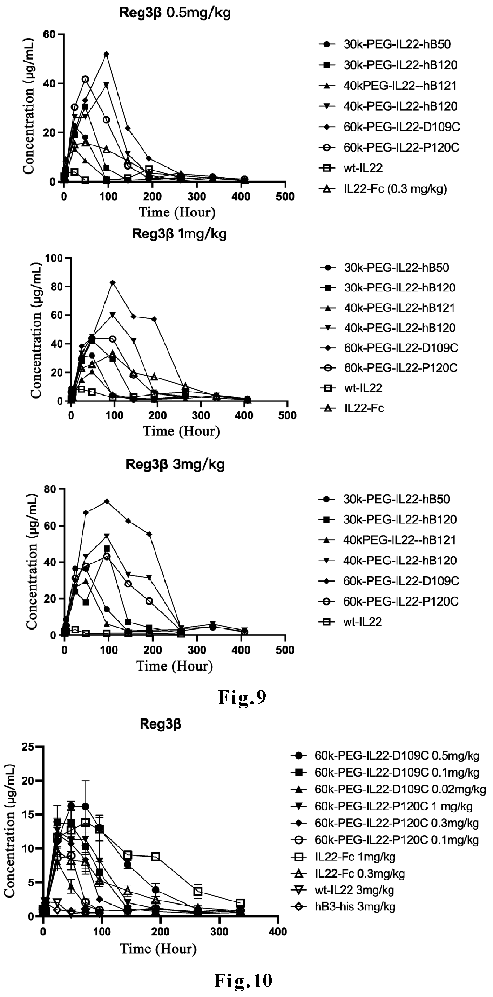

Approximately 40 µL of orbital blood was collected with anticoagulation on the day before dosing and at 4, 24, 48, 96, 144, 192, 264, 336, and 408 hours after dosing, followed by plasma separation. ELISA plates were coated overnight at 4°C with 1 µg/mL anti-Reg3β (Sino Biological, 51153-R065) dissolved in PBS. Mouse Reg3β (Sino Biological, 51153-M08H) was serially diluted to generate a positive control standard curve. Plasma samples were appropriately diluted to fall within the linear range of the standard curve. After blocking, samples were added and incubated for 2 hours. Incubation was then performed with the secondary antibody sheep anti-mouse Reg3β (R&D Systems, AF5110-SP) and HRP-conjugated antibody (HRP-Rabbit anti-sheep IgG H&L, Abcam, Ab97130). After final washing, tetramethylbenzidine peroxidase substrate (TMB, Surmodics, TMBS-1000-01) was added for color development for 10 minutes. The reaction was terminated with 2 M sulfuric acid, and absorbance was read at 450 nm with 630 nm as reference using a microplate reader. The original concentrations of Reg3β in each sample were calculated. Concentration versus time curves were plotted (Figure 9).

The results showed that all test candidates were able to activate Reg3β expression to a certain extent. wt IL-22 at different doses exhibited relatively weak activation of Reg3β, with no obvious dose-dependent enhancement. At similar (0.5 mg/kg vs. 0.3 mg/kg) or identical (1 mg/kg) doses, compared with IL-22-Fc, the test candidates 60K-PEG-IL22-D109C, 60K-PEG-IL22-P120C, and 40K-PEG-IL22-hB120 exhibited higher peak concentrations of Reg3β stimulation.

**Table 11. Peak plasma concentrations of Reg3β in mice after single administration of PEGylated IL22 mutants**

| | Mutant name (test candidate) | Reg3βCmax | Normalized Cmax ratio of Reg3β (relative to IL-22-Fc) |
|---|---|---|---|
| 0.5mg/k g | 30K-PEG-IL22-hB50 | 22.6 | 143% |
| | 30K-PEG-IL22-hB120 | 30.6 | 194% |
| | 40K-PEG-IL22-hB120 | 39.3 | 249% |
| | 40K-PEG-IL22-hB121 | 13.3 | 84% |
| | 60K-PEG-IL22-D109C | 52.0 | 329% |
| | 60K-PEG-IL22-P120C | 41.8 | 265% |
| | IL-22-Fc (0.3 mg/kg) | 15.8 | 100% |
| | wt IL-22 | 3.9 | 25% |
| 1mg/kg | 30K-PEG-IL22-hB50 | 31.9 | 97% |
| | 30K-PEG-IL22-hB120 | 42.3 | 128% |
| | 40K-PEG-IL22-hB120 | 60.0 | 182% |
| | 40K-PEG-IL22-hB121 | 20.7 | 63% |
| | 60K-PEG-IL22-D109C | 82.9 | 251% |
| | 60K-PEG-IL22-P120C | 43.9 | 133% |
| | IL-22-Fc | 33.0 | 100% |
| | wt IL-22 | 8.3 | 25% |
| 3mg/kg | 30K-PEG-IL22-hB50 | 36.5 | / |
| | 30K-PEG-IL22-hB120 | 47.4 | / |
| | 40K-PEG-IL22-hB120 | 54.2 | / |
| | 40K-PEG-IL22-hB121 | 29.9 | / |
| | 60K-PEG-IL22-D109C | 73.4 | / |
| | 60K-PEG-IL22-P120C | 43.3 | / |
| | IL-22-Fc | N.D | / |
| | wt IL-22 | 3.2 | / |

### 7. Mouse PD Preference Study (Reg3β and SAA)

Based on the *in vitro* preference results and preliminary PD screening results, the molecules 60k-PEG-IL22-D109C and 60k-PEG-IL22-P120C were selected. PD testing was performed in three mice per dose group at different dose levels according to the method described in Example 14, including the pharmacodynamic-related molecule Reg3β and the inflammation-related molecule SAA. The control was IL-22-Fc. Blood samples were collected before dosing and at 4, 24, 48, 72, 96, 144, 192, 264, and 336 hours after dosing. Plasma was separated using anticoagulated blood samples. For the control molecules wt IL-22 and hB3-his, samples were collected only up to 96 hours.

The detection method for Reg3β was performed according to Example 14, and the original concentration of Reg3β in each sample was calculated. Concentration versus time curves were plotted.

The detection method for SAA was as follows: a kit containing detecting antibody pairs and positive controls (Abcam, Ab216059) was used to detect SAA concentrations in appropriately diluted plasma samples. The assay was performed according to the manufacturer's instructions. Sa-HRP (Pierce 21126) was used for conjugation. After washing, tetramethylbenzidine peroxidase substrate (TMB, Surmodics, TMBS-1000-01) was added for color development for 10 minutes. The reaction was terminated with 2 M sulfuric acid, and the plate was read at 450 nm with 630 nm as the reference using a microplate reader. SAA was measured before dosing and at 24, 48, 72, 96, and 144 hours after dosing. The original SAA concentration in each sample was calculated. Concentration versus time curves were plotted (Figures 10-11).

The results of the mouse PD preference study showed that wt IL-22 and hB3-his stimulated significantly lower levels of Reg3β production in mice compared with the other test candidates. Under conditions where Reg3β Cmax was equivalent or superior among different dose levels of the candidate test candidates (e.g., 60K-PEG-IL22-D109C 0.1 mg/kg vs 60K-PEG-IL22-P120C 0.3 mg/kg vs IL-22-Fc 1 mg/kg), the site-specifically conjugated PEG-IL22 test candidates induced significantly lower SAA Cmax compared with IL-22-Fc, suggesting better non-proinflammatory preference, capable of maintaining organ-protective activity while significantly reducing pro-inflammatory effects.

**Table 12. Dosing information for the mouse PD preference study (Reg3β and SAA)**

| Serial Number | Mutant name (test candidate) | ROA | Dose (mg/kg) |
|---|---|---|---|
| 1 | 60K-PEG-IL22-P120C | s.c. | 1/0.3/0.1 |
| 2 | 60K-PEG-IL22-D109C | s.c. | 0.5/0.1/0.02 |
| 3 | IL-22-Fc | s.c. | 1/0.3 |
| 4 | hB3-his | i.p. | 3 |
| 5 | wt IL-22 | i.p | 3 |

### 8. Mouse PK

Male Balb/C mice (n = 4 per group) were administered a single subcutaneous injection at a dose of 1 mg/kg. Blood samples were collected before dosing and at 4 (no samples were collected at this time point for 60k-PEG-IL22-D109C and 60k-PEG-IL22-P120C), 8, 24, 24, 48, 72, 96, and 120 hours after dosing. Anticoagulated blood was used to prepare plasma.

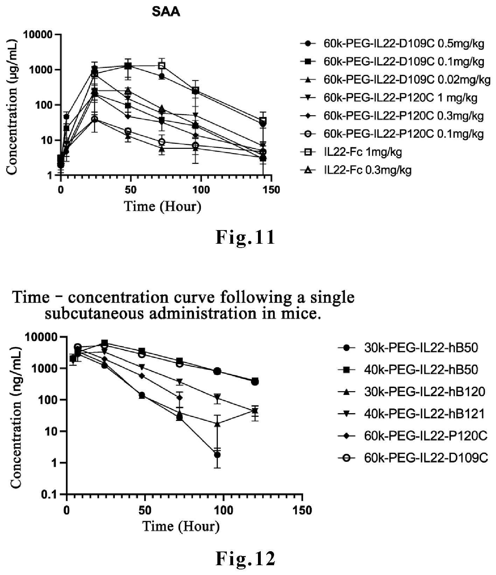

The concentrations of each test candidate in plasma were quantified by ELISA. Anti-IL22 Antibody (Sino Biological, 13059-MM24), diluted in PBS to 2 µg/mL, was used for coating. Standard curves of each test candidate and plasma samples were diluted to fall within the linear range of detection. Recombinant Anti-IL22 Antibody (Sino Biological, 13059-R034-H) was used for detection. The original concentration of each test candidate in the samples was calculated using a four-parameter fit of the standard curve for each test candidate. Pharmacokinetic parameters of each test candidate in mouse plasma were analyzed (Figure 12).

**Table 13. Dosing information for the mouse PK study**

| Mutant name (test candidate) | Dose | Mode of administration |
|---|---|---|
| 30k-PEG-IL22-hB50 | 1 mg/kg | s.c. |
| 40k-PEG-IL22-hB50 | 1 mg/kg | s.c. |
| 30k-PEG-IL22-hB120 | 1 mg/kg | s.c. |
| 40k-PEG-IL22-hB121 | 1 mg/kg | s.c. |
| 60k-PEG-IL22-P120C | 1 mg/kg | s.c. |
| 60k-PEG-IL22-D109C | 1 mg/kg | s.c. |

The pharmacokinetic parameters of each test candidate were analyzed as follows:

**Table 14. Pharmacokinetic parameters of each test candidate in mice**

| Mutant name (test candidate) | T1/2 (h) | Cmax (ng/mL) | AUClast (h*ng/mL) | MRT (h) |
|---|---|---|---|---|
| 30k-PEG-IL22-hB50 | 7.697538 | 2859.37 | 65366.62 | 16.01626 |
| 40k-PEG-IL22-hB50 | 22.81423 | 6458.657 | 322336.1 | 40.13081 |
| 30k-PEG-IL22-hB120 | 17.69736 | 3423.535 | 77617.96 | 17.13642 |
| 40k-PEG-IL22-hB121 | 14.92585 | 3355.216 | 142226.4 | 28.63004 |
| 60k-PEG-IL22-P120C | 11.62414 | 4184.95 | 105202.9 | 20.17036 |
| 60k-PEG-IL22-D109C | 21.59887 | 5460.625 | 301583.5 | 41.39287 |

### 9. Mouse DSS-Induced Colitis Efficacy Model

Balb/C mice were used to evaluate efficacy in a DSS-induced mouse acute colitis model. DSS refers to Dextran Sulfate Sodium Salt (MW: 36,000-50,000). When added to drinking water at an appropriate concentration, DSS disrupts intestinal epithelial cells and induces acute inflammatory bowel disease (IBD) in mice. Therefore, a 4% DSS-induced mouse IBD model was used to evaluate the potential therapeutic efficacy of the test candidates.

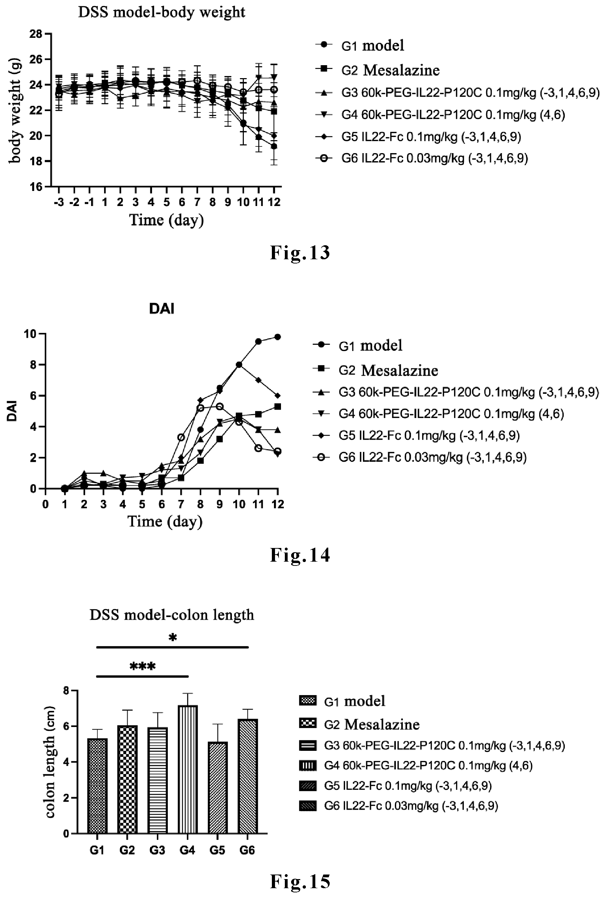

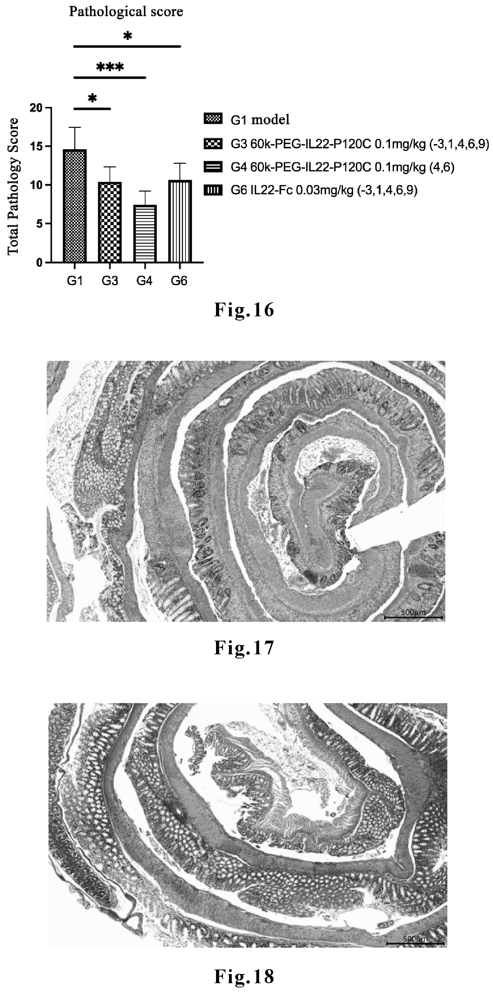

Male Balb/C mice were allowed free access to drinking water containing 4% DSS until the average body weight of the model group decreased by approximately 15%. Fresh drinking water was then provided, followed by a 2-day recovery period. Body weight and the severity of loose stool/hematochezia for each mouse were monitored throughout the study (Figure 13), and a composite Disease Activity Index (DAI) was evaluated (Figure 14). At the endpoint, the colon of each mouse was collected and its length measured (Figure 15). The colons were photographed, rolled into a Swiss-roll configuration, fixed in 4% paraformaldehyde, dehydrated, embedded in paraffin, sectioned, and subjected to H&E staining (Figures 17-18). Histological scoring was performed on tissue morphology(Figure 16).

The dosing regimen of the test candidates was as follows (the day of DSS modeling was defined as D1, the previous day as D-1, and so on).

**Table 15. Dosing information for the mouse DSS-induced colitis model study**

| Group | Number | Drug Information | Dose | Route of | Dosing |
|---|---|---|---|---|---|
| | | (Mutant Name) | (mg/kg) | Administration | Schedule |
| G1 | 6/♂ | - | - | - | - |
| G2 | 6/♂ | Mesalazine | 100 | p.o. | QD*10 |
| G3 | 6/♂ | 60K-PEG-IL22-P120C | 0.1 | s.c. | (-3, 1, 4, 6, 9) |
| G4 | 6/♂ | 60K-PEG-IL22-P120C | 0.1 | s.c. | (4, 6) |
| G5 | 6/♂ | IL-22-Fc | 0.1 | s.c. | (-3, 1, 4, 6, 9) |
| G6 | 6/♂ | IL-22-Fc | 0.03 | s.c. | (-3, 1, 4, 6, 9) |

**Table 16. DAI scoring criteria**

| Score | Body Weight Loss (%) | Stool Consistency | Fecal Blood |
|---|---|---|---|
| 0 | 0 | Normal | Normal |
| 1 | 1-5 | Soft Stool | Occult Blood |
| 2 | 6-10 | | |
| 3 | 11-20 | | |
| 4 | >20 | Loose Stool | Gross Blood in Stool |

Paraffin sections of colon tissues were subjected to H&E staining and histopathological analysis. According to the actual severity of the lesions, the following scoring criteria were used: minimal (1 point), mild (2 points), moderate (3 points), and severe (4 points). Lesion area (%) scoring criteria were defined as: ≥50% (4 points), 25%-50% (3 points), 10%-25% (2 points), and ≤10% (1 point). Inflammation depth scoring criteria were defined as: epithelium (1 point), lamina propria (2 points), and muscularis mucosae (3 points). No lesion was recorded as "-". The score was calculated by summing the scores from the above three dimensions.

In the model group (G1), histological sections showed relatively severe and extensive destruction of the colonic mucosal structure, loss of intestinal epithelium and crypts, as well as severe and widespread inflammatory cell infiltration and fibrous tissue hyperplasia in the mucosa and submucosa. In the 0.1 mg/kg 60k-PEG-IL22-P120C group (G4), the colon tissue morphology was relatively intact, the destruction of the colonic mucosal structure was not severe, and no significant inflammatory cell infiltration or fibrous tissue hyperplasia was observed.

The results demonstrated that the test candidate 60k-PEG-IL22-P120C exhibited clear efficacy in the mouse DSS-induced acute colitis model. Based on body weight, DAI, colon length, and histological scoring analyses, 0.1 mg/kg 60k-PEG-IL22-P120C (Group G4) showed superior efficacy compared with 0.03 mg/kg IL-22-Fc (Group G6).

### 10. Mouse Ear Injection Swelling Model

Balb/C mice were used for induction of ear skin inflammation. By repeated inner-ear injections of the test candidates, changes in ear swelling thickness after injection were measured to reflect the ability of the test candidates to induce local inflammatory responses in the skin.

On Days 0, 1, 2 and 4, 5 µL of PBS solution containing the indicated amount of each test candidate was injected into the inner side of both ears of each mouse. Ear thickness was measured daily using a micrometer, and the increase of ear thickness relative to Day 0 (delta thickness) on each day after injection was calculated.

**Table 17. Dosing information for the mouse ear swelling model**

| Mutant name (test candidate) | Dose (µg/ear) |
|---|---|
| 60k-PEG-IL22-P120C | 5 |
| | 1.6 |
| | 0.32 |
| IL-22-Fc | 1 |
| | 0.2 |
| wt IL-22 | 1 |

**Table 18. Peak increase in ear thickness after dosing in mice**

| Mutant Name (Test candidate) | Dose (µg/ear) | Mean peak increase in ear thickness (µm) |
|---|---|---|
| 60k-PEG-IL22-P120C | 5 | 89.7 |
| | 1.6 | 58.5 |
| | 0.32 | 51.8 |
| IL-22-Fc | 1 | 98.8 |
| | 0.2 | 69.7 |
| wt IL-22 | 1 | 34.8 |

The results showed that inner-ear injection of different doses of each test candidate in mice induced a dose-dependent increase in ear thickness (Figure 18). The equivalent dose of 60k-PEG-IL22-P120C required to induce a comparable overall increase in ear thickness was approximately 8-fold higher than that of IL-22-Fc (1.6 µg vs 0.2 µg). Combined with the efficacy dose results in mice (3-fold difference, 0.1 mg/kg vs 0.03 mg/kg), this reuslt suggests that, compared with IL-22-Fc, 60k-PEG-IL22-P120C has a lower potential risk of inducing skin toxicity.

The above descriptions represent only preferred embodiments of the present invention. It should be noted that, for those skilled in the art, various modifications and improvements may be made without departing from the principles of the present invention, and such modifications and improvements shall also fall within the scope of protection of the present invention.

## Claims

1. An IL-22 mutant, wherein at least one amino acid residue within positions 10 to 95 of the amino acid sequence set forth in SEQ ID NO: 1 is substituted with cysteine-PEG or p-acetyl-L-phenylalanine-PEG.

2. The IL-22 mutant according to claim 1, **characterized in that** the mutation site is selected from at least one of the following positions: position 10, position 12, position 15, position 16, position 17, position 18, position 21, position 22, position 64, position 72, position 75, position 76, position 79, position 83, position 84, position 86, position 87, position 88, position 91, or position 95.

3. The IL-22 mutant according to claim 1, **characterized in that** the average molecular weight of PEG ranges from 30kD to 80kD, preferably ranges from 30kD to 60kD.

4. The IL-22 mutant according to claim 1, **characterized in that** the mutant comprises at least one of the following mutations:
Asp at position 10 of the amino acid sequence set forth in SEQ ID NO: 1 is replaced with p-acetyl-L-phenylalanine-PEG30;
Ser at position 12 of the amino acid sequence set forth in SEQ ID NO: 1 is replaced with p-acetyl-L-phenylalanine-PEG30;
Gln at position 15 of the amino acid sequence set forth in SEQ ID NO: 1 is replaced with p-acetyl-L-phenylalanine-PEG30;
Gln at position 16 of the amino acid sequence set forth in SEQ ID NO: 1 is replaced with p-acetyl-L-phenylalanine-PEG30;
Pro at position 17 of the amino acid sequence set forth in SEQ ID NO: 1 is replaced with p-acetyl-L-phenylalanine-PEG30;
Pro at position 17 of the amino acid sequence set forth in SEQ ID NO: 1 is replaced with p-acetyl-L-phenylalanine-PEG40;
Pro at position 17 of the amino acid sequence set forth in SEQ ID NO: 1 is replaced with cysteine-PEG40;
Pro at position 17 of the amino acid sequence set forth in SEQ ID NO: 1 is replaced with cysteine-PEG60;
Pro at position 17 of the amino acid sequence set forth in SEQ ID NO: 1 is replaced with cysteine-PEG80;
Tyr at position 18 of the amino acid sequence set forth in SEQ ID NO: 1 is replaced with p-acetyl-L-phenylalanine-PEG30;
Asn at position 21 of the amino acid sequence set forth in SEQ ID NO: 1 is replaced with p-acetyl-L-phenylalanine-PEG30;
Arg at position 22 of the amino acid sequence set forth in SEQ ID NO: 1 is replaced with p-acetyl-L-phenylalanine-PEG30;
Asn at position 64 of the amino acid sequence set forth in SEQ ID NO: 1 is replaced with p-acetyl-L-phenylalanine-PEG30;
Phe at position 72 of the amino acid sequence set forth in SEQ ID NO: 1 is replaced with p-acetyl-L-phenylalanine-PEG30;
Ser at position 75 of the amino acid sequence set forth in SEQ ID NO: 1 is replaced with p-acetyl-L-phenylalanine-PEG30;
Asp at position 76 of the amino acid sequence set forth in SEQ ID NO: 1 is replaced with p-acetyl-L-phenylalanine-PEG30;
Asp at position 76 of the amino acid sequence set forth in SEQ ID NO: 1 is replaced with cysteine-PEG40;
Asp at position 76 of the amino acid sequence set forth in SEQ ID NO: 1 is replaced with cysteine-PEG60;
Asp at position 76 of the amino acid sequence set forth in SEQ ID NO: 1 is replaced with cysteine-PEG80;
Gln at position 79 of the amino acid sequence set forth in SEQ ID NO: 1 is replaced with p-acetyl-L-phenylalanine-PEG30;
Gln at position 83 of the amino acid sequence set forth in SEQ ID NO: 1 is replaced with p-acetyl-L-phenylalanine-PEG30;
Glu at position 84 of the amino acid sequence set forth in SEQ ID NO: 1 is replaced with p-acetyl-L-phenylalanine-PEG30;
Val at position 86 of the amino acid sequence set forth in SEQ ID NO: 1 is replaced with p-acetyl-L-phenylalanine-PEG30;
Pro at position 87 of the amino acid sequence set forth in SEQ ID NO: 1 is replaced with p-acetyl-L-phenylalanine-PEG30;
Pro at position 87 of the amino acid sequence set forth in SEQ ID NO: 1 is replaced with p-acetyl-L-phenylalanine-PEG40;
Pro at position 87 of the amino acid sequence set forth in SEQ ID NO: 1 is replaced with cysteine-PEG40;
Pro at position 87 of the amino acid sequence set forth in SEQ ID NO: 1 is replaced with cysteine-PEG60;
Pro at position 87 of the amino acid sequence set forth in SEQ ID NO: 1 is replaced with cysteine-PEG80;
Phe at position 88 of the amino acid sequence set forth in SEQ ID NO: 1 is replaced with p-acetyl-L-phenylalanine-PEG30;
Phe at position 88 of the amino acid sequence set forth in SEQ ID NO: 1 is replaced with p-acetyl-L-phenylalanine-PEG40;
Arg at position 91 of the amino acid sequence set forth in SEQ ID NO: 1 is replaced with p-acetyl-L-phenylalanine-PEG30;
Arg at position 95 of the amino acid sequence set forth in SEQ ID NO: 1 is replaced with p-acetyl-L-phenylalanine-PEG30.

5. A biological material selected from any one of the following items I)-V):
I) a nucleic acid encoding the IL-22 mutant as described above;
II) an expression cassette comprising the nucleic acids of I);
III) an expression vector comprising the nucleic acid of I) or the expression cassette of II);
IV) a host comprising the expression vector of III); and
V) a host in which the nucleic acid of I) or the expression cassette of II) is integrated into the genome.

6. A method for preparing the IL-22 mutant according to any one of claims 1 to 4, comprising:
replacing at least one amino acid within positions 10-95 of the amino acid sequence shown in SEQ ID NO:1 with p-acetyl-L-phenylalanine, and reacting the resulting protein with PEG-hydroxylamine to obtain the mutant according to any one of claims 1 to 4; or
replacing at least one amino acid within positions 10-95 of the amino acid sequence shown in SEQ ID NO:1 with cysteine, and reacting the resulting protein with PEG-maleimide to obtain the mutant according to any one of claims 1 to 4.

7. The method according to claim 6, **characterized in that** the catalyst for the reaction with PEG-hydroxylamine is ammonium chloride and acetylhydrazine.

8. A pharmaceutical composition comprising the IL-22 mutant according to any one of claims 1-4.

9. Use of the IL-22 mutant according to any one of claims 1 to 4 or the pharmaceutical composition according to claim 8 in the manufacture of a medicament for treating immune-related diseases.

10. The use according to claim 9, **characterized in that** the immune-related disease comprises at least one of rheumatoid arthritis, psoriasis and psoriatic arthritis, vitiligo, alopecia areata, dystrophic epidermolysis bullosa, systemic lupus erythematosus, insulin-dependent diabetes mellitus, diabetic foot ulcer, Crohn's disease, ulcerative colitis, atherosclerosis, hemolytic anemia, rheumatic fever, thyroiditis, myasthenia gravis, glomerulonephritis, pancreatitis, hepatitis, fatty liver disease, multiple sclerosis, or graft-versus-host disease.

11. A method for altering IL-22 receptor activation preference while reducing the toxicity of the IL-22 mutant, comprising:
substituting the amino acid at position 76 and/or position 87 of IL-22 as shown in SEQ ID NO:1 with p-acetyl-L-phenylalanine or cysteine; and
site-specifically PEGylating the substituted amino acid to obtain the IL-22 mutant.

12. The method according to claim 11, **characterized in that** the average molecular weight of the PEG ranges from 30kD to 80kD, preferably from 30kD to 60kD.
